# EUROPEAN PATENT APPLICATION

(11) **EP 4 840 873 A1**
(43) Date of publication of application: **18.11.2026**
(21) Application number: 26178796.4
(22) Date of filing: 14.05.2026
(51) Int. Cl.: C07K 5/02, A61K 38/00, A61P 35/00, C07K 5/103

(54) **LCP1 PEPTIDES WITH ANTITUMOUR ACTIVITY**

(30) Priority: 14.05.2025 IT 202500010846
(71) Applicant: Università degli Studi di Catania, 95131 Catania (IT)
(72) Inventor: Furneri, Pio Maria, 95131 Catania (IT); Fuochi, Virginia, 95131 Catania (IT); Fichera, Marco, 95131 Catania (IT); Rescifina, Antonio, 95131 Catania (IT); Floresta, Giuseppe, 95131 Catania (IT); Patamia, Vincenzo, 95131 Catania (IT); Zagni, Chiara, 95131 Catania (IT)
(74) Representative: Mola, Edoardo

(57) **Abstract**

The object of the present invention are polypeptide compounds of formula (**I**), in neutral form or in the form of salts, aggregates, complexes or solvates, which are able to inhibit the growth and development of tumor lines. Compositions including such peptide compounds in combination with at least one pharmacologically acceptable excipient, and at least one synthetic method for obtaining such compounds and/or compositions, are also the object of the present invention. The present invention also covers said peptide compounds of formula (**I**) for use as a medicament, in particular as an anticancer medicament for the treatment of carcinomas such as colon carcinoma, basal cell carcinoma and cervical carcinoma.

## Description

### Technical field

The subject of the present invention are peptide compounds of formula **(I)** capable of inhibiting the growth and development of tumor lines. Compositions comprising these peptide compounds in combination with at least one excipient and at least one synthetic method for obtaining such compounds and/or compositions are also the subject of this invention. The present invention also covers peptide compounds with formula **(I)** for use as an anticancer medicament, preferably in the treatment of colon carcinoma, basal cell carcinoma and cervical carcinoma.

### Known art

Substances of natural origin are considered as promising alternatives to common conventional therapies (Yuan et al., 2016). Among the macromolecules that play an essential role in cell growth and development are the components of the bacterial cell wall, i.e. peptidoglycans (PGs) (Wheeler et al., 2014). The PG polymer is composed of N-acetylmuramic and N-acetylglucosamine sugar chains alternating with cross-linked peptide chains containing D- and L-amino acids. Overall, PG is made up of a dynamic and complex structure that plays an essential role in bacterial physiology and in maintaining the shape and integrity of the cell itself. PG also acts as an interface between the bacterium and its environment. Recently, attention to it has increased thanks to its promising pharmacological activities, comprising anticancer (He et al., 2017; Wang et al., 2018). Numerous studies have highlighted the effectiveness of probiotic bacteria in the treatment of several medical conditions, comprising colorectal cancer (CRC) (Ishikawa et al., 2005; Uccello et al., 2012; Chong, 2014; Fong et al., 2020). In this context, many studies have shown that some lactobacilli species, such as *Lactobacillus casei,* have a remarkable antitumor activity both *in-vivo* and *in-vitro* (Kato et al., 1981; Yasutake et al., 1984). Several lactobacilli are thought to be beneficial to health, as they maintain gut microbial balance, protect against invasion by pathogenic microorganisms, and activate the host's innate immune response (Veiga et al., 2020).

Extracts and fragments of peptidoglycan can induce apoptosis in several cell cancer lines (Troll et al., 2009; Vazquez-Sanchez et al., 2014; Wang et al., 2018; Patra et al., 2022). The main tumor suppressor protein is p53, which induces cell death by apoptosis in response to various stress conditions (Aubrey et al., 2018). p53 inactivation is the most common defect in human tumors. p53 is negatively regulated by the oncoprotein MDM2 (murine double minute 2). MDM2 is an E3 ubiquitin ligase that targets the degradation of the tumor suppressor p53 and inhibits the expression of p53-related genes. Since MDM2 and p53 have been found to be part of a self-regulatory feedback loop, a new approach for cancer treatment is to stimulate p53 by inhibiting its interaction with MDM2 (Wu et al., 1993).

In the scientific article **[1],** the cytotoxic activity of the peptidoglycan of *Lactobacillus casei* against various tumor cells was reported. On the basis of the known chemical formula of the basic peptide component of the peptide complex of *L. casei,* the peptide (hereinafter referred to as LCP1) has been synthesized: 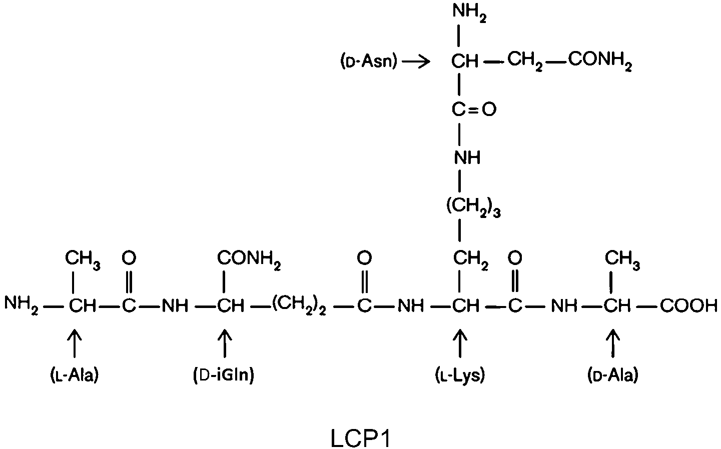 and its cytotoxicity has been tested against cancer and normal cells. The synthetic peptide was found to be able to impair the entire metabolism of cultured cancer cells and restore the apoptotic process. In contrast, normal cells appeared to be stimulated rather than inhibited by the peptide, while primary mouse embryonic fibroblasts behaved similarly to cancer cells.

In EP0545352A1, pharmaceutical compositions containing *Lactobacillus casei* peptidoglycan in pharmacologically active quantities, suitable for use in anticancer therapy, have been described. The paper highlights that treatment with a suitable peptidoglycan of *Lactobacillus casei* causes the inhibition of the activity of cancer cells, thanks to a mechanism of direct toxicity that acts as an inhibitor on these cells.

Given these encouraging results, it is evident that there is a need to explore the surrounding chemical space in search of peptide compounds that may express antitumor activities that improve those described in the literature.

Unless otherwise stated, the contents of this paragraph are to be understood as an integral part of the Detailed Description below.

### Summary of the invention

It is an object of the present invention a compound of formula (I) 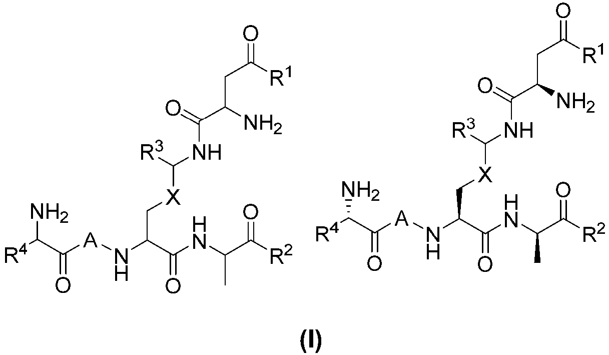 wherein
**X** = -CH₂-, -CH₂CH₂-;
**R¹** = -OH, -NH₂;
**R²** = -OH, 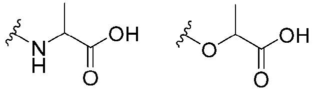
**R³** = -H, -COOH;
**R⁴** = -H, -CH₃; 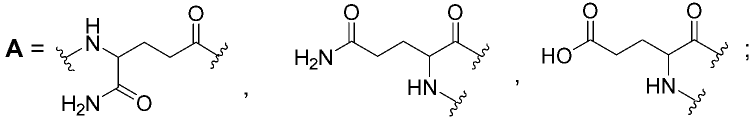 and
or a pharmaceutically acceptable salt, zwitterion, stereoisomer or solvate, and considering the LCP1 compound excluded from the scope of protection.

It is an object of the present invention also a compound with a general formula **(II):** 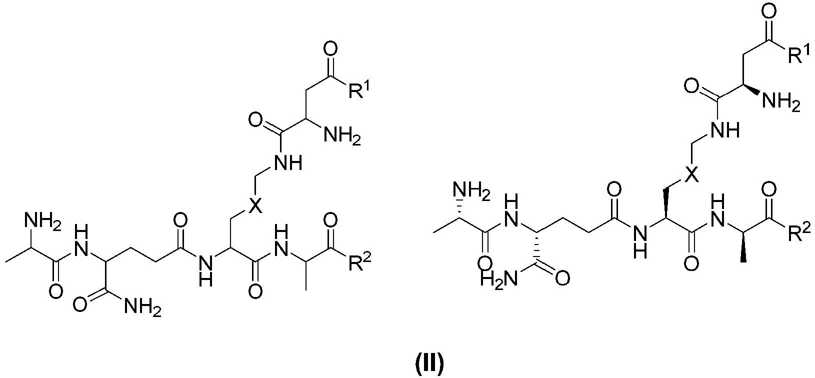 wherein
**X** = -CH₂-, -CH₂CH₂-;
**R¹** = -OH, -NH₂;
**R²** = -OH, 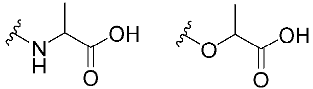
or its pharmaceutically acceptable salt, zwitterion, stereoisomer, and considering the **LCP1** compound excluded from the scope of protection.

At least one method for the synthesis of a compound of formula (II) is also an object of the present invention.

Another object of the present invention is a composition comprising at least one compound of formula (I) or (II) and at least one pharmaceutically acceptable excipient.

The present invention also covers said composition formulated in the form of powder, granules, flakes, tablets, lozenges, briquettes (tiles of agglomerated material), pills, liquid, semi-solid, gelled in the form of creams, pomades, lotions, gels, ointments, macroemulsions, microemulsions, nanoemulsions, serums, balms, colloids, solutions, liquids, suspensions, dispersions, spray formulations, suppositories, ovules.

Furthermore, compositions formulated for an administering form chosen from topical, oral, pulmonary, intranasal, buccal, rectal, vaginal, parenteral, intravenous, intracutaneous, subcutaneous, intramuscular, ophthalmic administration are an object of the present invention.

Finally, at least one compound of formula (I) or (II) or a combination thereof for use as a medicament, preferably as a medicament for the treatment of cancer, most preferably for the treatment of colon carcinoma, basal cell carcinoma and cervical carcinoma, is covered by the present invention.

### Brief description of the Figures

The invention will be described below in at least one preferred embodiment for the sole purpose of explanation and not limiting the scope of the present invention with the help of the figures in the annex, wherein:
- **Figure 1****:** Left: Best docking conformation of LCP1 within the MDM2 site groove; right: Best docking conformation of LCP2 within the MDM2 site.
- **Figure 2****:** Left: Best LCP3 docking conformation within the MDM2 site; Right: Best LCP1Ala docking conformation within the MDM2 site.
- **Figure 3****:** Left: Best docking conformation of LCP2Lat within the MDM2 site; Right: Best docking conformation of LCP3Ala within the MDM2 site.
- **Figure 4****:** Left: Best docking conformation of LCP1 within hexokinase site 2; right: Best docking conformation of LCP2 within hexokinase site 2.
- **Figure 5****:** Left: Best docking conformation of LCP3 within hexokinase site 2; right: Best docking conformation of LCP1Ala within hexokinase site 2.
- **Figure 6****:** Left: Best docking conformation of LCP2Lat within hexokinase site 2; right: Best docking conformation of LCP3Ala within hexokinase site 2.
- **Figure 7:** a) 24-hour anti proliferative activity of LCP1, LCP2 and LCP3 on Caco-2 cell line; b) Cytotoxic activity of LCP1, LCP2 and LCP3 on Caco-2 cell line. C+ represents positive control.
- Figure 8**:** a) 24-hour anti proliferative activity of LCP1, LCP2 and LCP3 on HCT-116 cell line; b) Cytotoxic activity of LCP1, LCP2 and LCP3 on HCT-116 cell line. C+ represents positive control.
- Figure 9**:** a) 24-hour anti proliferative activity of LCP1, LCP2 and LCP3 on the CCD-18Co cell line; b) Cytotoxic activity of LCP1, LCP2 and LCP3 on the CCD-18Co cell line. C+ represents positive control.
- **Figure 10:** a) Treatment of the HCT-8 cell line at 48h with LCP1; b) Treatment of the HCT-8 cell line at 48h with LCP2; c) Treatment of the HCT-8 cell line at 48h with LCP3
- **Figure 11:** a) Treatment of the HCT-116 cell line at 48h with LCP1; b) Treatment of the HCT-116 cell line at 48h with LPC2; c) Treatment of the HCT-116 cell line at 48h with LCP3.
- **Figure 12:** a) Treatment of the Caco-2 cell line for 48h with LCP1; b) Treatment of the Caco-2 cell line for 48h with LPC2; c) Treatment of the Caco-2 cell line at 48h with LCP3.
- **Figure 13:** a) MTT test of intestinal organoids treated for 24h with LCP1, LCP2 and LCP3; b) MTT test of intestinal tumors treated for 24h with LCP1, LCP2 and LCP3.
- **Figure 14****:** LCP1Glu, left, best docking conformation within the MDM2 groove; right, best docking conformation within the hexokinase 2 groove.
- **Figure 15****:** LCP2Glu, left, best docking conformation within the MDM2 groove; right, best docking conformation within the hexokinase 2 groove.
- **Figure 16****:** LCP3Glu, left, best docking conformation within the MDM2 groove; right, best docking conformation within the hexokinase 2 groove.
- **Figure 17****:** LCP1bGlu, left, best docking conformation within the MDM2 groove; right, best docking conformation within the hexokinase 2 groove.
- **Figure 18****:** LCP2bGlu, left, best docking conformation within the MDM2 groove; right, best docking conformation within the hexokinase 2 groove.
- **Figure 19****:** LCP3bGlu, left, best docking conformation within the MDM2 groove; right, best docking conformation within the hexokinase 2 groove.
- **Figure 20****:** LCP1Gln, left, best docking conformation within the MDM2 groove; right, best docking conformation within the hexokinase 2 groove.
- **Figure 21****:** LCP2GIn, left, best docking conformation within the MDM2 groove; right, best docking conformation within the hexokinase 2 groove.
- **Figure 22****:** LCP3GIn, left, best docking conformation within the MDM2 groove; right, best docking conformation within the hexokinase 2 groove.
- **Figure 23****:** LCP1bGln, left, best docking conformation within the MDM2 groove; right, best docking conformation within the hexokinase 2 groove.
- **Figure 24****:** LCP2bGln, left, best docking conformation within the MDM2 groove; right, best docking conformation within the hexokinase 2 groove.
- **Figure 25****:** LCP3bGln, left, best docking conformation within the MDM2 groove; right, best docking conformation within the hexokinase 2 groove.

### Definitions

- In this document, formulas (I) and (II) comprise all equivalent structural representations, regardless of how stereochemistry is graphically illustrated.
- In this document, the terms "approximately" or "about" as used herein when referring to a measurable value such as a quantity, a time duration, and the like, are intended to encompass variations of ±20%, ±10%, ±5%, ±1%, or ±0.1% from the specified value, wherein such variations are appropriate to perform the methods described.

- In this document, the terms, "comprises," "includes," "has," "having," "contains," "containing," "characterized by," or any other variation of these terms, are intended to cover a non-exclusive inclusion, subject to any limitations expressly stated. For instance, a composition, mixture, process, or method that comprises a list of elements is not necessarily limited to these elements but may comprise other elements not expressly listed or inherent in that composition, mixture, process, or method.
- In this document, the quantity of each element constituting the composition according to the present invention expressed as a percentage range is to be understood as comprising the values at the edge of the range itself. So, if a component is between 10% and 20%, the extremes of the range are included in the range.
- In this document, percentages are understood as % by weight (w/w%) unless otherwise indicated.
- In this document, the term "aggregate" or "molecular aggregate" refers to a set of molecules comprising at least one compound with a general formula (I), said molecules gathered together to form more complex and structured molecular structures (e.g., dimeric complexes, trimeric complexes).

### Detailed Description

The subject of the present invention are peptide compounds of formula **(I)** with antitumor activity.

These organic compounds have a peptide structural backbone that is electively modified to emphasize the growth and development of inhibitory activity of tumor lines by means of a direct toxicity mechanism.

Starting from a molecule known in the state of the art (LCP1) [1], 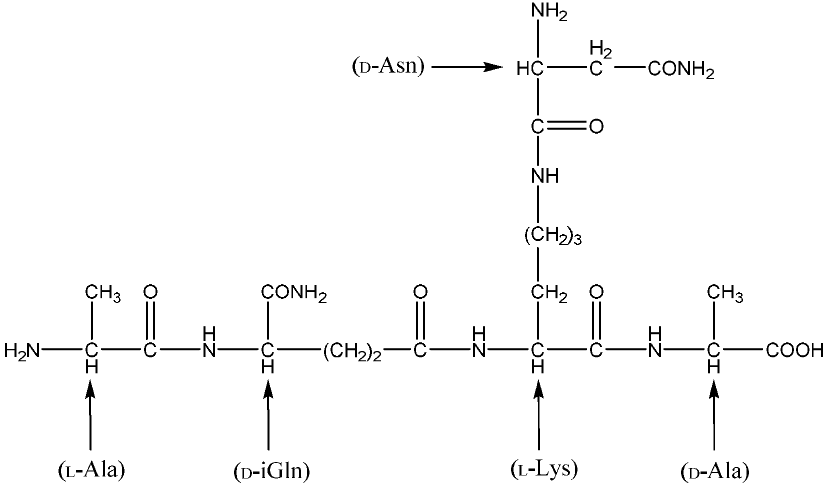 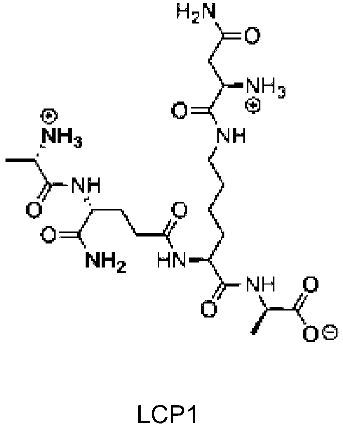 the inventors of the present invention conducted a Hit-to-Lead (H2L) search for the identification of a set of compounds with significantly increased antitumor activity compared to the starting substrate (LCP1).

The present invention concerns compounds of general formula **(I):** 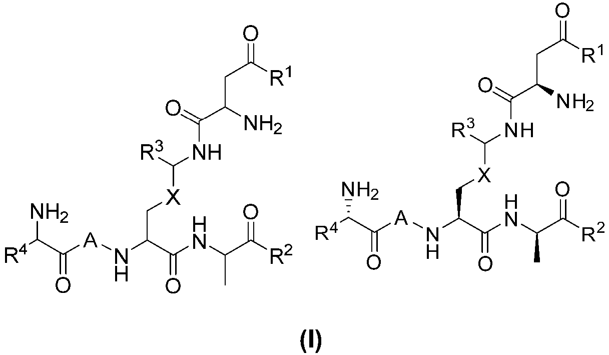 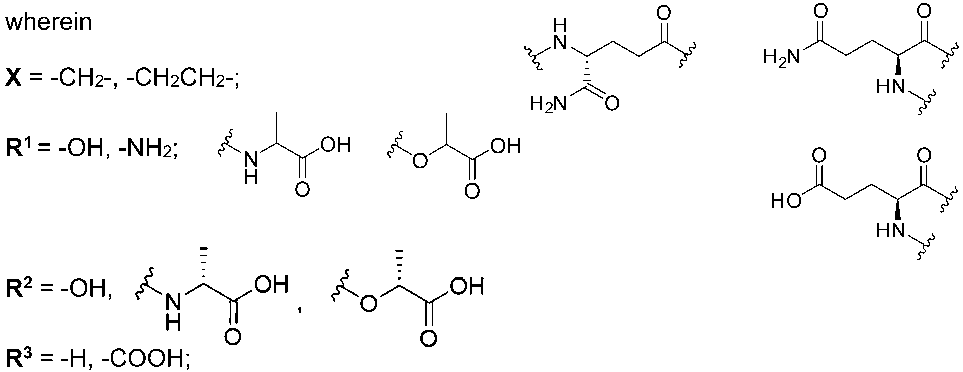 **R⁴ =** ₃; 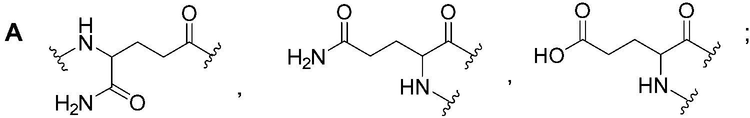 and considering **LCP1,** as such or in saline, neutral or zwitterionic form, excluded from the scope of protection.

Preferred, are the general formula compounds **(II):** 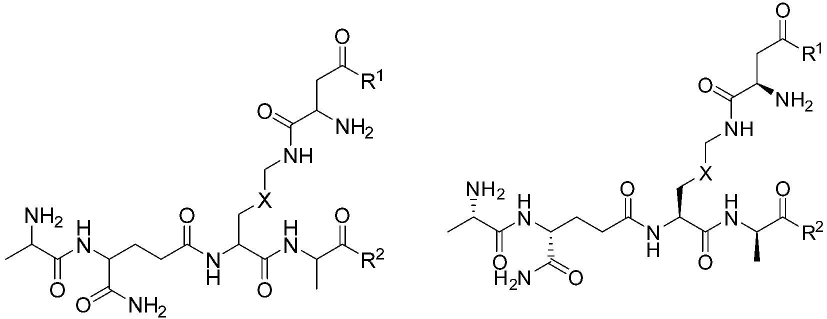 wherein
**X** = -CH₂-, -CH₂CH₂-; 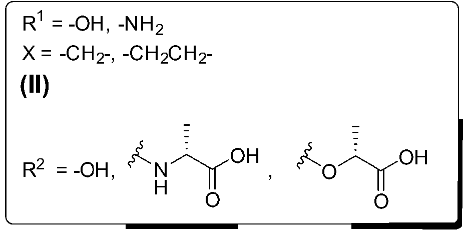
**R¹** = -OH, -NH₂; 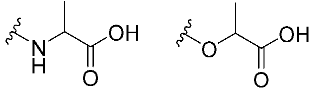
**R² = -OH,** 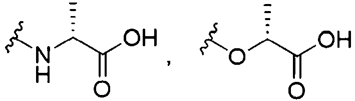 and
**LCP1** is excluded from the scope of protection.

The most preferred are the compounds shown in table 1, *i.e.* LCP2, LCP3, LCP1Ala, LCP2Lat and LCP3Ala, etc.

**Table 1.**

| | | |
|---|---|---|
| LCP2 | Lys) | (D-Ala) |
| | 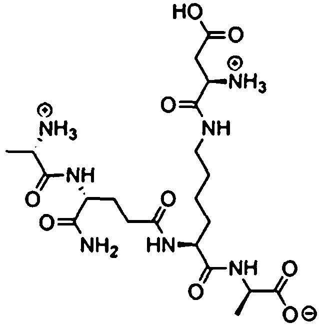 | |
| LCP3 | | |
| | 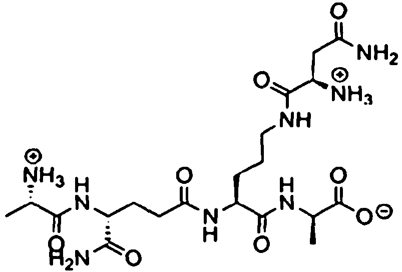 | |
| LCP1Ala SEQ ID NO: 1 | 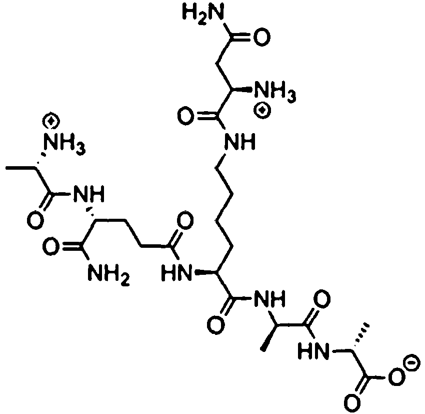 | |
| LCP2Lat | 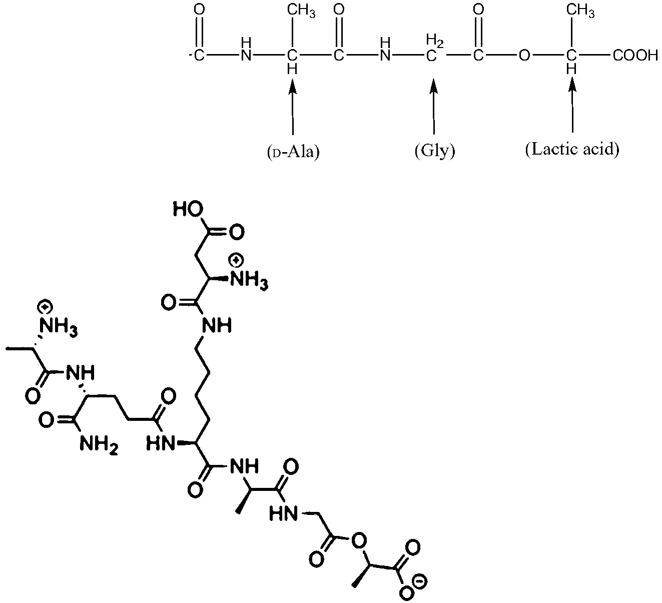 | |
| LCP3Ala | | |
| | 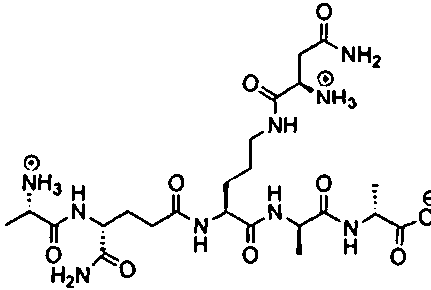 | |
| LCP1Gln | | |
| LCP2Gln | | |
| LCP3Gln | 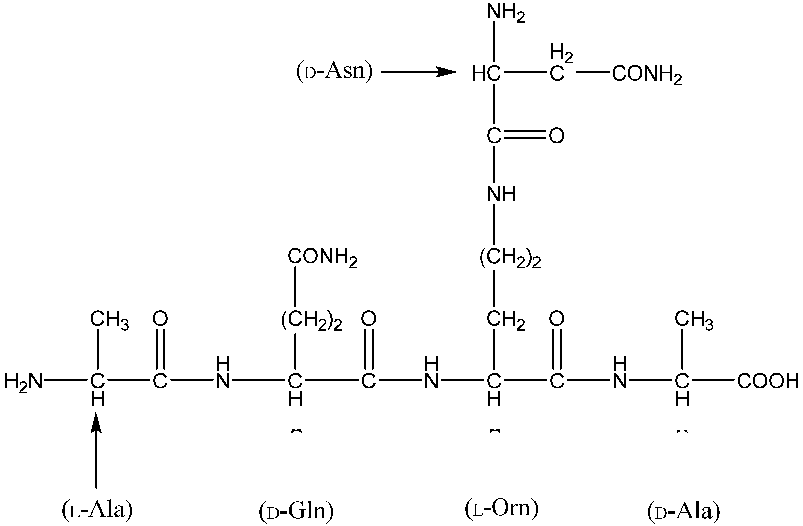 | |
| LCP1bGln | 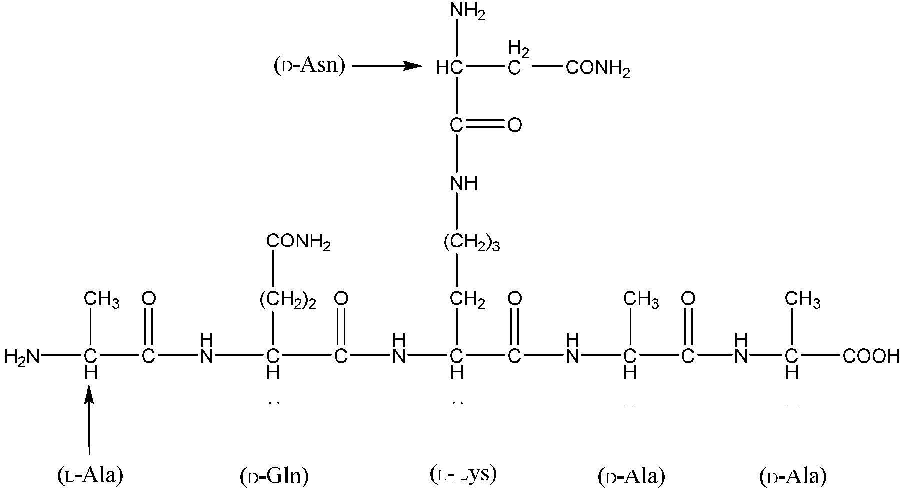 | |
| LCP2bGln | 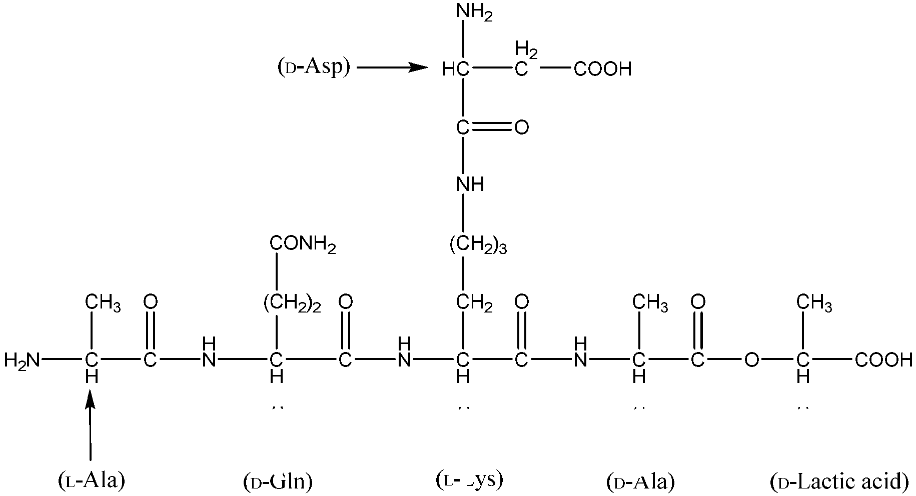 | |
| LCP3bGln | 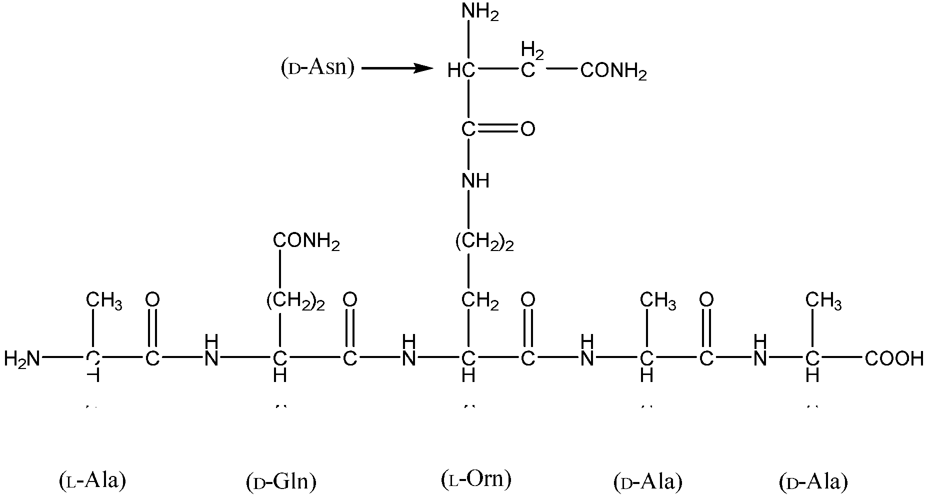 | |
| LCPlGlu | 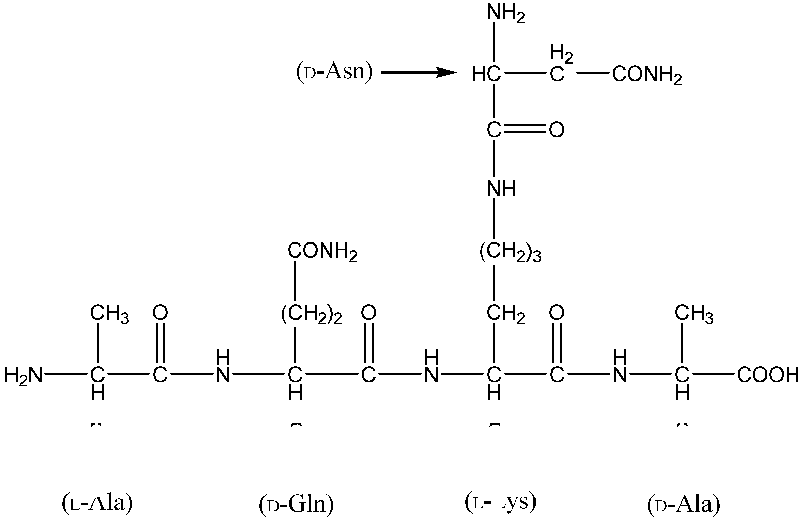 | |
| LCP2Glu | 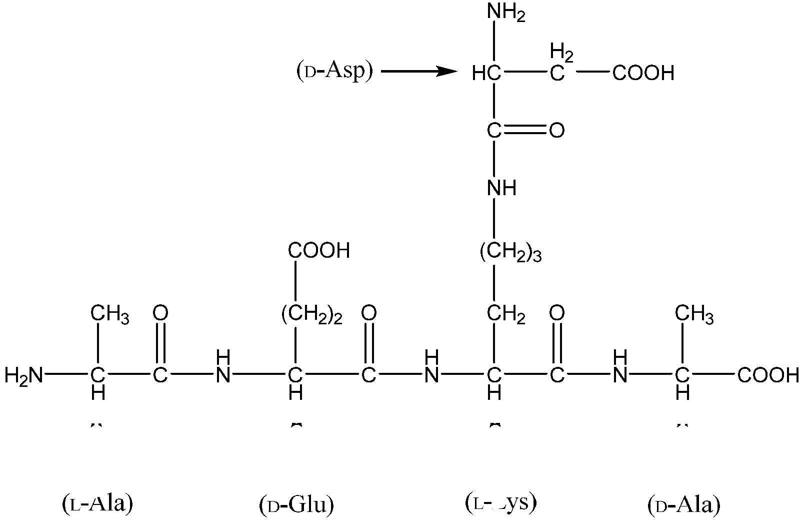 | |
| LCP3Glu | 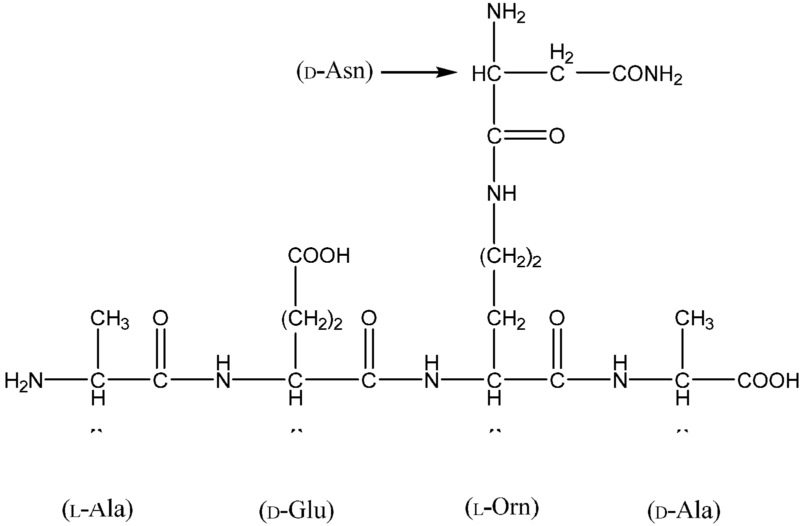 | |
| LCP1bGlu | 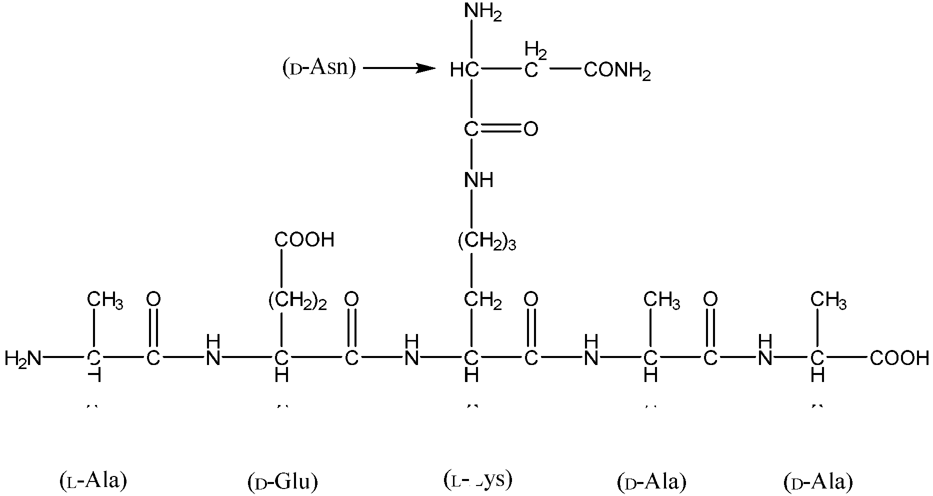 | |
| LCP2bGlu | 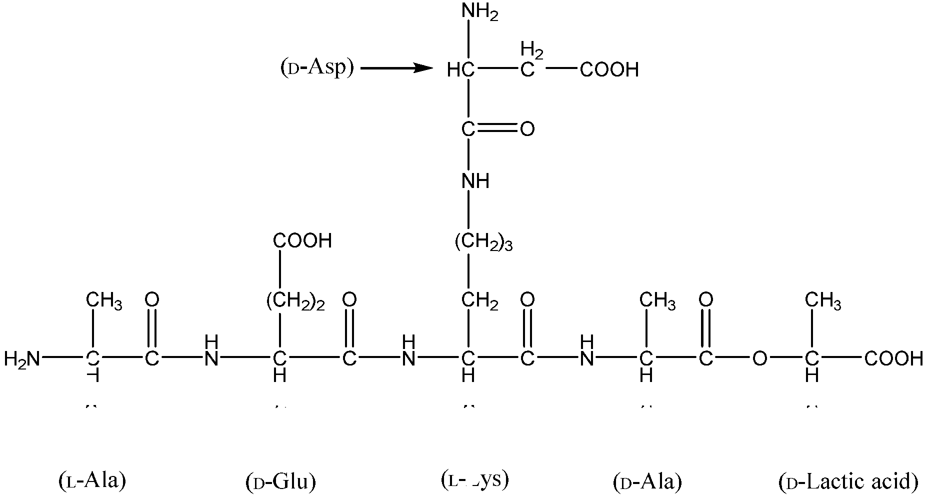 | |
| LCP3bGlu | 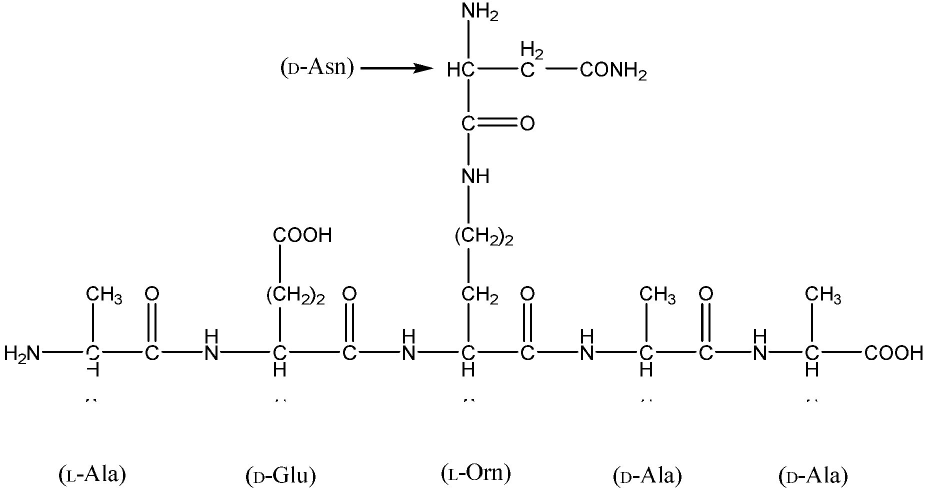 | |

In particular,
a LCP1Ala is identified by the sequence SEQ ID NO: 1 = AXKAA.

The other peptides in Table 1 can be identified by the following sequences:
- LCP2, SEQ ID NO: 2 = AXKA
- LCP3, SEQ ID NO: 3 = AXXA
- LCP2Lat, SEQ ID NO: 4 = AXKAX
- LCP3Ala, SEQ ID NO: 5 = AXXAA
- LCP1Gln, SEQ ID NO: 6 = AQKNA
- LCP2GIn, SE ID NO: 7 = AQKDA
- LCP3GIn, SEQ ID NO: 8 = AQXA
- LCP1bGln, SEQ ID NO: 9 = AQKNAA
- LCP2bGIn, SEQ ID NO: 10 = AQKDAX
- LCP3bGIn, SEQ ID NO: 11 = AQXAA
- LCP1, SEQ ID NO: 12 = AQKNA
- LCP2Glu, SEQ ID NO: 13 = AEKDA
- LCP3Glu, SEQ ID NO: 14 = AEXA
- LCP1bGlu, SEQ ID NO: 15 = AEKNAA
- LCP2bGlu, SEQ ID NO: 16 = AEKDAX
- LCP3bGlu, SEQ ID NO: 17 = AEXAA

Compounds of formula **(I)** and **(II)** can be prepared in the form of single stereoisomers, raceme or resolved mixtures (i.e., optically active), diasteroisomeric mixtures, distinct polymorphic forms or mixtures/combinations thereof.

Formula **(I)** and **(II)** compounds may be used alone, in combination with each other or in combination with other ingredients and/or excipients and/or active ingredients in pharmaceutically acceptable compositions.

Compounds of formula **(I)** and **(II)** can be prepared and/or used in neutral or (poly)ionic form (i.e., as a pure or mixed salt), e.g. as cation, anion, polycation, polyanion (depending on the pH, for instance), as zwitterion, molecular complex, molecular aggregate, solvate and combinations thereof.

The derived salts of the compounds of formula **(I)** and **(II)** which are the subject of the present invention are to be understood as saline forms prepared by acid-base reaction with acidic or basic, organic or inorganic, non-toxic and pharmaceutically acceptable agents.

In a preferred embodiment, compounds of formula **(I)** and **(II)** according to the invention are prepared in (poly)cationic form as pure or mixed salts, preferably pure.

These compounds in (poly)cationic form can have at least one organic or inorganic anion as a counterion.

These compounds in (poly)cationic form, by way of example but not limited to, may have as a counterion at least one anion chosen from chloride (Cl⁻), sulphate (SO₄²⁻ ), acetate (CH₃COO⁻), phosphate (PO₄³⁻), nitrate (NO₃⁻), bicarbonate (HCO₃⁻), citrate (C₆H₅O₇³⁻), trifluoroacetate (CF₃COO⁻)-

Particularly preferred as anionic counterions are acetate (CH₃COO⁻) and trifluoroacetate (CF₃COO⁻)

In another embodiment, compounds of formula **(I)** and **(II)** according to the invention are prepared in (poly)anionic form as pure or mixed salts, preferably pure.

Said compounds in (poly)anionic form can have organic or inorganic cations as counterion.

Salts of formula **(I)** and **(II) compounds** derived from pharmaceutically acceptable non-toxic organic bases comprise but are not limited to: primary, secondary, and tertiary amine salts, substituted amines comprising naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine and tromethamine.

### Compositions

The present invention also comprises **compositions** comprising:
- at least one compound with formula **(I)** or **(II)** as an active ingredient or a derivative thereof (*i.e.,* a pure or mixed salt, complex, aggregate, solvated, zwitterion) that is pharmaceutically acceptable; and
- at least one pharmaceutically acceptable excipient.

Compositions according to the invention can be obtained by conventional pharmaceutical compounding techniques, known to a technician in the field.

According to the invention, the at least one excipient is to be considered as a substance or mixture of substances with no therapeutic effect, which is added to the pharmaceutical formulation together with the active ingredient to facilitate the preparation, administration, and overall efficacy of the drug. Excipients play important roles in drug stability, dosage, absorption, and distribution.

Said at least one excipient can be chosen by way of example but not limited to from the group comprising:
- pharmaceutical vehicles (carriers);
- solubilizers;
- buffering systems;
- stabilizers;
- thickeners; and
- gelling agents.

The pharmaceutical vehicle according to the invention can be chosen by way of example from the group comprising water, purified water, deionized water, saline, glycerol, natural oils, glucose and combinations thereof.

The solubilizer according to the invention can be chosen by way of example from the group comprising propylene glycol, ethanol, PEG, glycerol, nonionic surfactants (e.g., tween, span), amphiphilic surfactants, sorbitol and combinations thereof.

The buffering system according to the invention can be chosen by way of example from the group comprising phosphate solution, citrate/citric acid solution, acetic acid/acetate solution, bicarbonate/carbonate solution. The industry expert will be able to choose the appropriate buffering system depending on the desired pH.

The stabilizer according to the invention can be chosen by way of example but not limited to from the group comprising:
- antioxidants, preferably ascorbic acid, sodium metabisulfite, parabens;
- chelating agents, preferably EDTA;
- protein stabilizers, preferably lecithin, albumin;

The at least one antioxidant in the composition according to the invention allows the therapeutic potential of said composition to be preserved by protecting at least one peptide compound of formula **(I)** or **(II)** from oxidative degradation.

The at least one chelating agent of the composition according to the invention is capable of capturing/trapping metal cations present in said composition, inhibiting/limiting/preventing the degradation of compounds of formula **(I)** or **(II)** catalysed by said cations.

The thickener according to the invention can be chosen by way of example but not limited to from the group comprising xanthan gum, polyacrylic acid, arabic gum, guar gum, hydroxypropyl methylcellulose (HPMC), methylcellulose (MC), sodium alginate, carrageenan, and combinations thereof. The at least one thickener that can be optionally implemented in the composition of the invention allows, depending on the application needs, to alter the physical consistency of the formulation and to modulate the speed/ease of release of the active ingredient *(i.e.,* of the at least one compound of formula **(I)** or **(II)).**

The gelling agent according to the invention can be chosen as a non-limiting example from the group comprising agar-agar, pectin, and combinations thereof.

### Formulations

The composition according to the invention can be formulated in solid state in the form of powder, granules, flakes, tablets, tabs, briquettes (tiles of agglomerated material), pills.

The composition according to the invention can be formulated in liquid, semi-solid, gelled state in the form of creams, ointments, lotions, gels, unguents, macroemulsions, microemulsions, nanoemulsions, serums, balms, colloids, solutions, liquids, suspensions, dispersions, spray formulations.

The composition according to the invention can be further formulated for a mediated release by medical devices, preferably by means of plasters, bandages, fabric cloths, wipes, masks, aerosols.

The various formulations are within the reach of the technician in the field and described in the literature.

### Routes of administration

Formula **(I)** and **(II)** compounds, their derivatives (i.e., saline, zwitterion, complex, aggregate, solvate forms) and their pharmaceutically acceptable compositions can be prepared in the form of formulations suitable for a choice of topical, oral, pulmonary (e.g., intranasal or buccal inhalation), rectal or vaginal administration.

Parenteral, intravenous, intracutaneous, subcutaneous, intramuscular, ophthalmic administrations may also be envisaged.

The route of administration and the most suitable amount of active substance in each specific case will depend on the age and sex of the patient, the nature and severity of the conditions to be treated and will be indicated by the treating physician.

In a preferred embodiment, compounds having formula **(I)** and **(II),** their derivatives and their pharmaceutically acceptable compositions, are administered orally, preferably by discrete units such as capsules, sachets or tablets each containing a predetermined amount of the active ingredient, such as a powder, granules, solution, or suspension in an aqueous liquid, a non-aqueous liquid, an oil-in-water liquid emulsion, or a water-in-oil liquid emulsion.

In another preferred embodiment, formula **(I)** and **(II)** compounds, their derivatives, and their pharmaceutically acceptable compositions, are administered topically, preferably as gel or ointment.

In the case of skin cancers, the transdermal patch is further preferred as a medical device for the controlled release of formula **(I)** and **(II)** compounds, their derivatives and their pharmaceutically acceptable compositions.

In another preferred embodiment, formula **(I)** and **(II)** compounds, their derivatives and their pharmaceutically acceptable compositions are administered intranasally, preferably delivered by spray, nasal solutions or aerosols, e.g., from pressurized packs or nebulizers.

In another form of implementation, compounds of formula **(I) and (II),** their derivatives and their pharmaceutically acceptable compositions are dispensed as powders, preferably with the aid of a powder inhalation device by insufflation.

In another preferred embodiment, compounds of formula **(I)** and **(II),** their derivatives and their pharmaceutically acceptable compositions are administered rectally or vaginally, preferably by suppositories or ovules.

### Posology

The extent of the prophylactic or therapeutic dose of composition to be administered, in particular the dose of active ingredient (i.e., formula **(I)** and **(II)** compounds, their respective saline, zwitterionic, complexed forms, aggregates, solvates) will depend on various factors, comprising the route of administration, the frequency and duration of treatment, severity of the pathological condition to be treated, the possible use of other drugs, as well as the age, weight and sex of the patient, from the patient's response but always according to the assessment of the attending physician.

### In silico study

The preferred compounds LCP1Ala, LCP2, LCP2Lat, LCP3, LCP3Ala were initially identified by *in silico* structure-activity relationship (SAR) analysis using and optimizing the organic skeleton of LCP1.

Before proceeding with the *in-vitro* analysis, computational molecular docking studies were conducted to evaluate the affinity of LCP1, LCP2, LCP3, LCP1Ala, LCP2Lat and LCP3Ala compounds with the following target proteins:
1. **MDM2** receptor (1YCR); and
2. Hexokinase 2 (5HG1), hereinafter simply **E2.**

As regards the interaction with the binding groove of **MDM2,** the values of binding free energy (ΔG_{leg}, [kcal/mol]) and inhibition constant (Kᵢ, [µM]) are shown in Table 2.

**Table 2**

| | ΔG_{leg} (kcal/mol) | Kᵢ (µM) |
|---|---|---|
| LCP1 | -5.7 | 65 |
| LCP2 | -5.8 | 55 |
| LCP3 | -5.7 | 64 |
| LCP1Ala | -5.4 | 109 |
| LCP2Lat | -5.5 | 92 |
| LCP3Ala | -5.9 | 43 |
| LCP1Gln | -6.8 | 9 |
| LCP2Gln | -7.3 | 4 |
| LCP3Gln | -7.4 | 4 |
| LCP1bGln | -7.2 | 5 |
| LCP2bGln | -7.4 | 4 |
| LCP3bGln | -8.0 | 1 |
| LCPlGlu | -7.2 | 5 |
| LCP2Glu | -7.2 | 5 |
| LCP3Glu | -7.1 | 6 |
| LCP1bGlu | -6.7 | 12 |
| LCP2bGlu | -7.5 | 3 |
| LCP3bGlu | -7.1 | 6 |

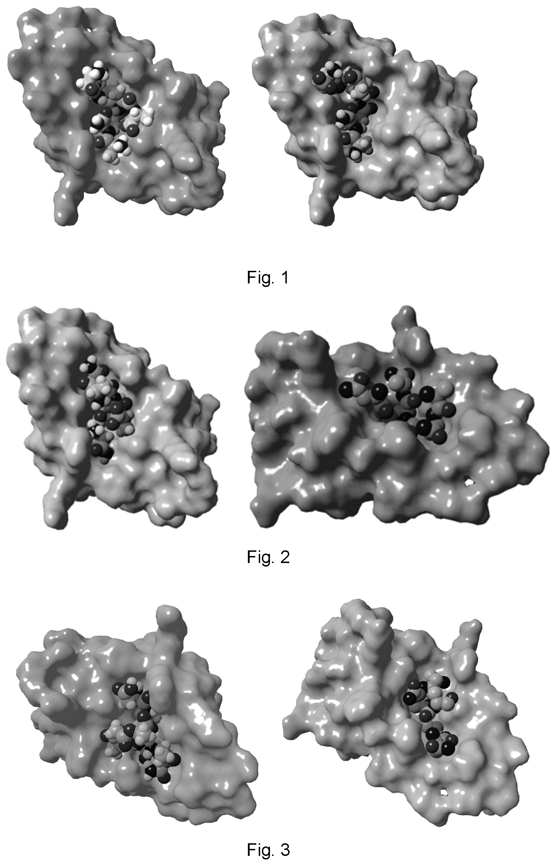

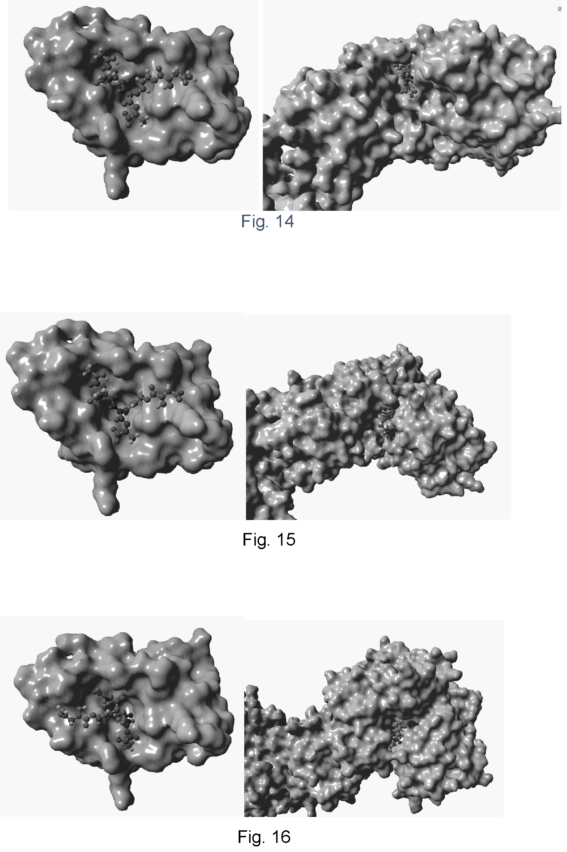

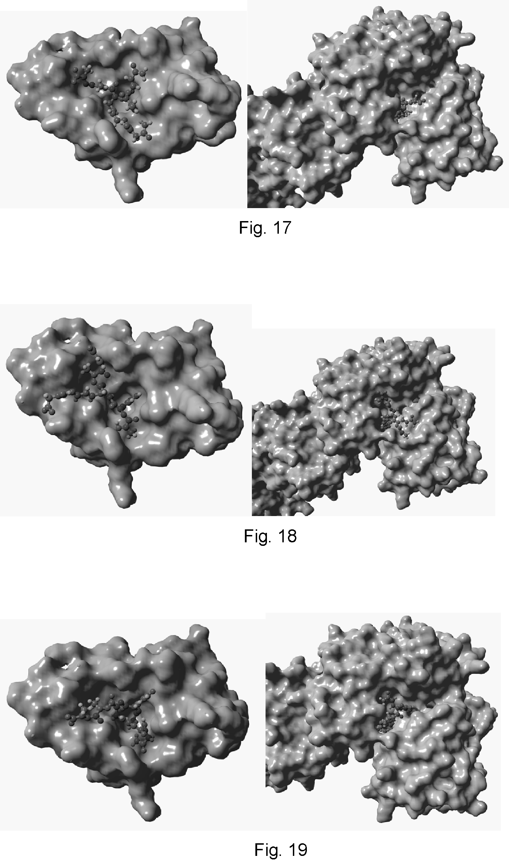

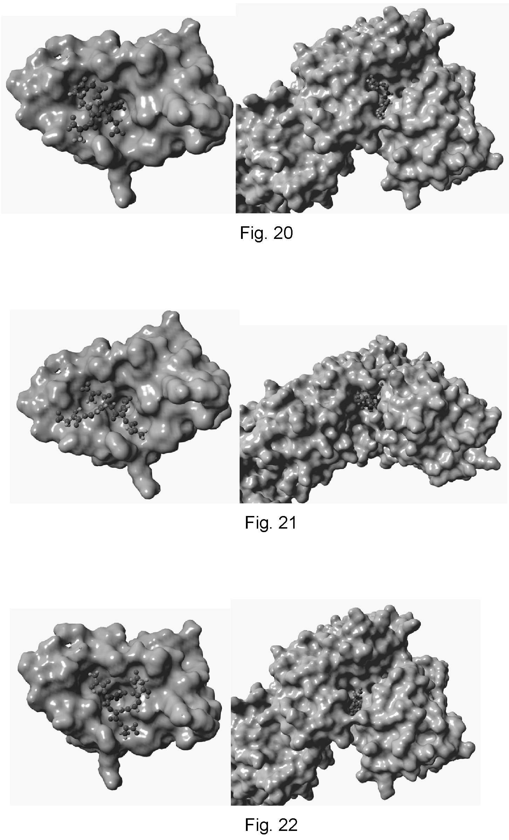

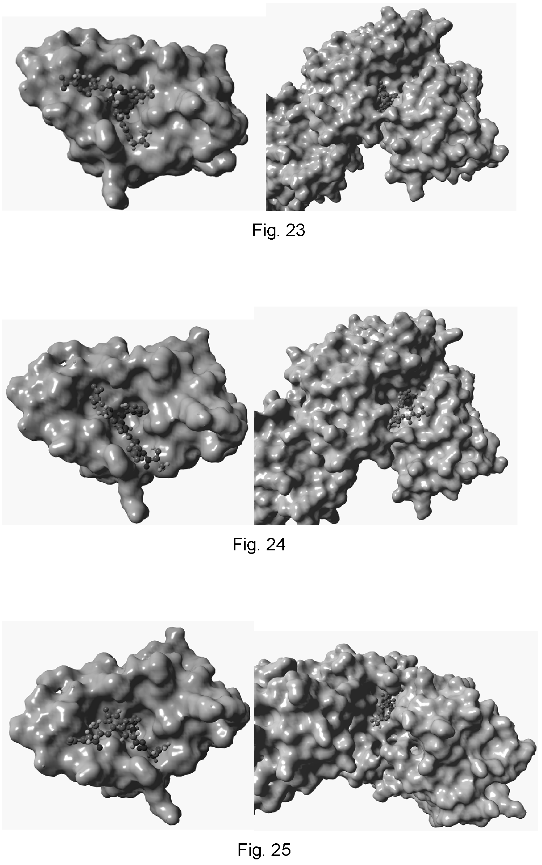

In particular,
- **LCP1** showed key interactions with MDM2 on residues of Met 50, Leu 54, Phe 55, Leu 57, Gly 58, Ile 61, Met 62, Tyr 67, Gln 72, His 73, Val 75, Phe 91, Val 93, His 96, Ile 99, Tyr 100, Ile 103 (Figure 1-left);
- **LCP2,** similarly to LCP1, interacted with MDM2 involving the residues Met 50, Leu 54, Leu 57, Gly 58, Ile 61, Met 62, Tyr 67, Gln 72, His 73, Val 75, Phe 91, Val 93, His 96, Ile 99, Tyr 100, Ile 103 (Figure 1-right);
- **LCP3** engaged MDM2 residues Leu 54, Leu 57, Gly 58, Ile 61, Met 62, Tyr 67, Gln 72, His 73, Val 75, Phe 91, Val 93, His 96, Ile 99, Tyr 100, Ile 103 (Figure 2-left);
- **LCP1Ala** interacted with Lys 51, Leu 54, Phe 55, Leu 57, Gly 58, Ile 61, Met 62, Tyr 67, Gln 72, His 73, Val 75, Phe 91, Val 93, His 96, Ile 99, Tyr 100, Ile 103 (Figure 2-right). The greater binding efficacy found for LCP1Ala is attributable to additional interactions such as those with Lys51 and Phe91;
- **LCP2Lat** showed interactions with Met 50, Leu 54, Phe 55, Leu 57, Gly 58, Gln 59, Ile 61, Met 62, Tyr 67, Gln 72, His 73, Val 75, Phe 91, Val 93, His 96, Ile 99, Tyr 100, Ile 103 of MDM2 (Figure 3-left);
- **LCP3Ala** showed better binding affinity for MDM2 than LCP1Ala and LCP3Ala, interacting with Leu 54, Phe 55, Leu 57, Gly 58, Ile 61, Met 62, Tyr 67, Gln 72, His 73, Val 75, Phe 91, Val 93, Lys 94, His 96, Ile 99, Tyr 100, Ile 103. (Figure 3-right).
- **LCP1Gln** showed interactions with Leu 54, Leu 57, Gly 58, Ile 61, Met 62, Tyr 67, Gln 72, His 73, Val 75, Phe 86, Phe 91, Val 93, Lys 94, His 96, Ile 99, Ile 103 (Figure 20-left)
- **LCP2GIn** showed interactions with Leu 54, Leu 57, Gly 58, Ile 61, Met 62, Tyr 67, Gln 71, Gln 72, His 73, Val 75, Phe 91, Val 93, Lys 94, His 96, Ile 99, Tyr 100, Ile 103 (Figure 21-left)
- **LCP3GIn** showed interactions with Lys 51, Leu 54, Phe 55, Leu 57, Gly 58, Gln 59, Ile 61, Met 62, Tyr 67, Gln 72, His 73, Phe 91, Val 93, His 96, Ile 99, Tyr 100, Ile 103 (Figure 22-left)
- **LCP1bGln** showed interactions with Met 50, Leu 54, Phe 55, Leu 57, Gly 58, Ile 61, Met 62, Gln 72, His 73, Phe 86, Phe 91, Val 93, Lys 94, His 96, Ile 99, Tyr 100, Ile 103 (Figure 23-left)
- **LCP2bGln** showed interactions with Leu 54, Leu 57, Gly 58, Ile 61, Met 62, Tyr 67, Gln 72, His 73, Val 75, Phe 91, Val 93, His 96, Ile 99, Tyr 100, Ile 103 (Figure 24-left)
- **LCP3bGln** showed interactions with Met 50, Leu 54, Leu 57, Gly 58, Ile 61, Met 62, Tyr 67, Gln 71, Gln 72, His 73, Val 75, Phe 86, Phe 91, Val 93, Lys 94, His 96, Ile 99, Tyr 100, Ile 103 (Figure 25-left)
- **LCP1Glu** showed interactions with Lys 51, Glu 52, Leu 54, Phe 55, Gly 58, Ile 61, Met 62, Tyr 67, Gln 72, His 73, Val 75, Val 93, His 96, Ile 99, Tyr 100 (Figure 14-left)
- **LCP2Glu** showed interactions with Lys 51, Glu 52, Leu 54, Phe 55, Leu 57, Gly 58, Ile 61, Met 62, Tyr 67, Gln 72, Val 75, Val 93, His 96, Ile 99, Tyr 100, Ile 103 (Figure 15-left)
- **LCP3Glu** showed interactions with Leu 54, Leu 57, Gly 58, Ile 61, Met 62, Tyr 67, Gln 71, Gln 72, His 73, Val 75, Phe 86, Phe 91, Val 93, Lys 94, His 96, Ile 99, Tyr 100, Ile 103 (Figure 16-left)
- **LCP1bGlu** showed interactions with Leu 54, Phe 55, Leu 57, Gly 58, Ile 61, Met 62, Tyr 67, Gln 72, Val 75, Phe 91, Val 93, His 96, Ile 99, Tyr 100, Ile 103 (Figure 17-left)
- **LCP2bGlu** showed interactions with Leu 54, Leu 57, Gly 58, Ile 61, Met 62, Tyr 67, Gln 71, Gln 72, His 73, Val 75, Val 93, Lys 94, His 96, Ile 99, Tyr 100, Ile 103 (Figure 18-left)
- **LCP3bGlu** showed interactions with Leu 54, Phe 55, Leu 57, Gly 58, Ile 61, Met 62, Gln 72, His 73, Phe 86, Phe 91, Val 93, Lys 94, His 96, Ile 99, Ile 103 (Figure 19-left)

On the other hand, with regard to the binding of the compounds of the invention with the active site of **E2,** the values of the binding free energy (ΔG_{leg}, [kcal/mol]) and the inhibition constant (Kᵢ [µM]) are shown in Table 3.

**Table 3**

| | ΔG_{leg} (kcal/mol) | Kᵢ (µM) |
|---|---|---|
| LCP1 | -7.5 | 3 |
| LCP2 | -7.5 | 2.8 |
| LCP3 | -7.6 | 2.8 |
| LCP1Ala | -8.1 | 1.03 |
| LCP2Lat | -8.4 | 0.6 |
| LCP3Ala | -7.8 | 1.93 |
| LCP1Gln | -9.6 | 0.08 |
| LCP2Gln | -10.0 | 0.04 |
| LCP3Gln | -10.5 | 0.02 |
| LCP1bGln | -9.7 | 0.07 |
| LCP2bGln | -9.3 | 0.16 |
| LCP3bGln | -8.4 | 0.72 |
| LCPlGlu | -9.5 | 0.11 |
| LCP2Glu | -9.5 | 0.11 |
| LCP3Glu | -10.0 | 0.04 |
| LCP1bGlu | -9.8 | 0.07 |
| LCP2bGlu | -8.4 | 0.73 |
| LCP3bGlu | -9.3 | 0.15 |

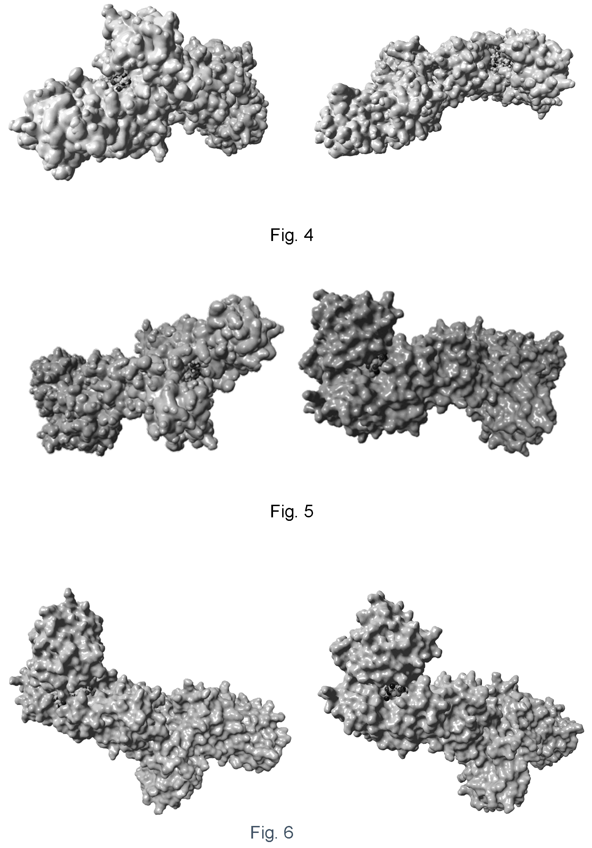

- **LCP1** showed key interactions with E2 on residues of Asp 84, Leu 85, Gly 86, Thr 88, Asn 89, Thr 153, Phe 154, Ser 155, Asn 208, Asp 209, Ile 229, Gly 231, Thr 232, Gly 233, Asn 287 shown in Figure 4-left;
- **LCP2** showed interactions with the amino acid residues Gly 534, Gly 535, Thr 536, Asn 537, Ile 562, Met 567, Thr 620, Lys 621, Gly 622, Phe 623, Gly 679, Thr 680, Gly 681, Leu 734, Asn 735, Gln 739, Glu 742, Lys 743, Gly 747, Met 748, Tyr 749, Thr 863 (Figure 4-right);
- **LCP3,** induced interactions with residues Asp 84, Gly 86, Thr 88, Asn 89, Arg 91, Ser 155, Asp 209, Thr 213, Ile 229, Val 230, Gly231, Thr 232, Gly 233, Asp 413, Gly 414, Ser 415, Lys 418, Lys 419, Glu 446, Asp 447, Gly 448, Ser 449, Gly 450 (Figure 5-left);
- **LCP1Ala** showed interactions with Pro 508, Lys 510, Ser 603, Phe 604, Pro 605, Leu 617, Lys 618, Thr 620, Lys 621, Asn 656, Asp 657, Thr 658, Ile 677, Thr 680, Gly 681, Ser 682, Asn 683, Glu 708, Ala 711, Leu 734, Asn 735, Lys 738, Gln 739, Glu 742, Tyr 749 as shown in Figure 5-right;
- **LCP2Lat** showed key interactions with residues Pro 508, Lys 510, Leu 512, Ser 603, Phe 604, Pro 605, Leu 617, Lys 618, Thr 620, Lys 621, Asn 656, Asp 657, Thr 658, Thr 680, Gly 681, Ser 682, Asn 683, Glu 708, Ala 711, Asp 714, Leu 734, Asn 735, Lys 738, Gln 739, Glu 742, Lys 743, Gly 747, Met 748, Tyr 749 (Figure 6-left);
- **LCP3Ala** showed interactions with the residues Pro 508, Val 509, Lys 510, Leu 512, Ser 603, Phe 604, Pro 605, Leu 617, Lys 618, Thr 620, Lys 621, Asn 656, Asp 657, Thr 680, Gly 681, Ser 682, Asn 683, Glu 708, Ala 711, Leu 734, Asn 735, Lys 738, Gln 739, Glu 742, Tyr 749. (Figure 6-right).
- **LCP1Gln** showed interactions with Pro 508, Lys 510, Pro 605, Leu 617, Lys 618, Thr 620, Lys 621, Gly 681, Ser 682, Asn 683, Glu 708, Ala 711, Asp 714, Asn 735, Lys 738, Gln 739, Glu 742, Tyr 749 (Figure 20-right)
- **LCP2GIn** showed interactions with Ser 603, Phe 604, Pro 605, Thr 620, Lys 621, Asn 656, Asp 657, Thr 658, Ile 677, Thr 680, Gly 681, Ser 682, Asn 683, Glu 708, Ser 733, Leu 734, Asn 735, Lys 738, Gln 739, Glu 742, Lys 743, Met 748, Tyr 749, Arg 779 (Figure 21-right)
- **LCP3GIn** showed interactions with Ser 603, Phe 604, Pro 605, Thr 620, Lys 621, Asn 656, Asp 657, Thr 658, Ile 677, Thr 680, Gly 681, Ser 682, Asn 683, Glu 708, Ser 733, Leu 734, Asn 735, Lys 738, Gln 739, Glu 742, Lys 743, Met 748, Tyr 749 (Figure 22-right)
- LCP1bGln showed interactions with Pro 508, Lys 510, Ser 603, Phe 604, Pro 605, Leu 617, Lys 618, Thr 620, Lys 621, Asn 656, Asp 657, Thr 658, Ile 677, Thr 680, Gly 681, Ser 682, Asn 683, Glu 708, Ala 711, Asp 714, Leu 734, Asn 735, Lys 738, Gln 739, Glu 742, Met 748, Tyr 749 (Figure 23-right)
- **LCP2bGln** showed interactions with Lys 510, Leu 512, Pro 605, Leu 617, Lys 618, Thr 620, Lys 621, Gly 681, Glu 708, Leu 734, Asn 735, Lys 738, Gln 739, Glu 742, Tyr 749 (Figure 24-right)
- **LCP3bGln** showed interactions with Ser 603, Phe 604, Pro 605, Lys 618, Trp 619, Thr 620, Lys 621, Asn 656, Asp 657, Thr 680, Gly 681, Ser 682, Asn 683, Glu 708, Leu 734, Asn 735, Lys 738, Gln 739, Glu 742, Lys 743, Gly 747, Met 748, Tyr 749 (Figure 25-right)
- **LCP1Glu** showed interactions with Ser 603, Phe 604, Pro 605, Thr 620, Lys 621, Gly 622, Asn 656, Asp 657, Ile 677, Thr 680, Gly 681, Ser 682, Asn 683, Glu 708, Ser 733, Leu 734, Asn 735, Lys 738, Gln 739, Glu 742, Lys 743, Met 748, Tyr 749, Arg 779 (Figure 14-right)
- **LCP2Glu** showed interactions with Ser 603, Phe 604, Pro 605, Thr 620, Lys 621, Gly 622, Asn 656, Asp 657, Thr 680, Gly 681, Ser 682, Asn 683, Glu 708, Ser 733, Leu 734, Asn 735, Lys 738, Gln 739, Glu 742, Lys 743, Met 748, Tyr 749, Arg 779 (Figure 15-right)
- **LCP3Glu** showed interactions with Pro 508, Lys 510, Phe 604, Pro 605, Lys 618, Thr 620, Lys 621, Asn 656, Ile 677, Thr 680, Gly 681, Ser 682, Asn 683, Glu 708, Gly 710, Ala 711, Ser 733, Leu 734, Asn 735, Lys 738, Gln 739, Glu 742, Tyr 749 (Figure 16-right)
- **LCP1bGlu** showed interactions with Ser 603, Phe 604, Pro 605, Leu 617, Lys 618, Thr 620, Lys 621, Asn 656, Asp 657, Thr 658, Ile 677, Thr 680, Gly 681, Ser 682, Asn 683, Glu 708, Ala 711, Ser 733, Leu 734, Asn 735, Lys 738, Gln 739, Glu 742, Lys 743, Tyr 749 (Figure 17-right)
- **LCP2bGlu** showed interactions with Ala 505, Ser 506, Ala 507, Pro 508, Lys 510, Ser 603, Phe 604, Pro 605, Ile 615, Leu 616, Leu 617, Lys 618, Thr 620, Lys 621, Glu 629, Gly 630, Glu 708, Ala 711, Asp 714, Lys 738, Gln 739, Glu 742 (Figure 18-right)
- **LCP3bGlu** showed interactions with Pro 508, Lys 510, Leu 512, Ser 603, Phe 604, Pro 605, Cys 606, Leu 617, Lys 618, Trp 619, Thr 620, Lys 621, Asn 656, Asp 657, Gly 681, Ser 682, Asn 683, Glu 708, Ala 711, Asp 714, Leu 734, Asn 735, Lys 738, Gln 739, Glu 742, Lys 743 (Figure 19-right)

As evident from the tables above, the lower *(in silico)* values of ΔG_{leg} and Kᵢ shown by peptides against E2 compared to MDM2 demonstrate a better binding affinity for hexokinase 2 (E2). Advantageously, structural modifications made to LCP1 and LCP3 resulted in peptide compounds with increased binding strength.

Precisely with regard to the inhibition of E2, the compounds according to the invention and in particular LCP1Ala and LCP2Lat, showed surprisingly lower Kᵢ values than the compound of known technique LCP1 demonstrating a better efficacy of inhibition of the protein receptor.

### In-vitro studies

*Evaluation of cytotoxicity and antiproliferative activity.*

Given the surprising activity values predicted by computational simulations, some of the aforementioned compounds were synthesized and tested *in-vitro* for the evaluation of antitumor properties.

Experimental evaluation of the peptides LCP2 and LCP3, along with the known art peptide LCP1 (used for comparison), showed significant effects in various biological systems, comprising cell lines and organoid cultures.

Cytotoxicity and antiproliferative activity tests were initially performed on a wide range of cell lines, both tumor and non-tumor.

The cytotoxic activity of the compounds according to the invention was evaluated by means of the MTT assay (known to an expert in the field and detailed in the Examples section).

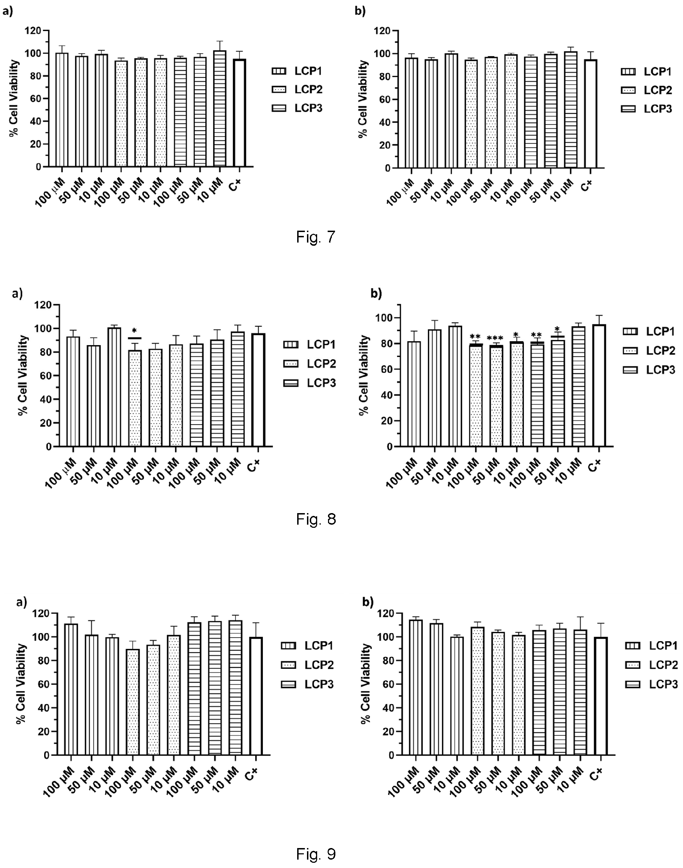

MTT assay results (Figures 7-9) provided insight into the antiproliferative and cytotoxic effects of LCP1, LCP2, and LCP3 peptides on three cell lines: Caco-2, HCT-116, and CCD-18Co.

No significant antiproliferative or cytotoxic activity was observed in the Caco-2 cell line for any of the peptides, indicating a lack of reactivity in this model (Figures 7a and 7b).

In the HCT-116 colorectal cancer cell line, LCP2 alone demonstrated anti proliferative activity, observed at the highest tested concentration of 100 µM (Figure 8a). Cytotoxicity testing, however, revealed more pronounced antitumor effects: LCP2 showed cytotoxic activity even at the lowest concentration tested (10 µM), with effects intensifying at higher doses (Figure 8b). LCP3 showed cytotoxic effects at intermediate (50 µM) and high (100 µM) concentrations, but no activity at 10 µM. In contrast, LCP1 showed no antiproliferative or cytotoxic effect in this cell line at all concentrations tested.

In the CCD-18Co non-tumor fibroblast cell line, no significant antiproliferative or cytotoxic effects were observed for any of the peptides, confirming their selective activity against tumor cells (Figures 9a and 9b).

These results underscore the potential of LCP2 as a strong candidate for anticancer applications, due to its efficacy in inhibiting cancer cell viability, particularly at lower concentrations. LCP3 also showed potential, although activity is limited to higher concentrations, while LCP1 demonstrated no therapeutic relevance in this setting.

The cytotoxic activity of the compounds according to the invention, also evaluated by MTT assay and indicated as the percentage of inhibition of the viability of tumor cell lines, was further investigated.

The results, expressed as % of cell viability inhibition ±SEM, are summarized in Table 4 while the specifications on the execution of the tests are reported in the Examples section.

**Table 4**

| **Cell lines** | **LCP1** | **LCP2** | **LCP3** |
|---|---|---|---|
| | 100 µM | 100 µM | 100 µM |
| CCD18Co | -14.5±1.5 | -8.4±0.9 | -5.6±0.6 |
| PDOs | -43.4±8.2 | -15.0±1.5 | -61.9±14.1 |
| HCT-8 | -14.0±4.1 | 29.0±2.9 | 30.8±6. |
| HCT-116 | 13.8±1.4 | 18.3±1.2 | 16.7±1.4 |
| HeLa | -10.6±0.7 | 27.9±2.4 | 14.5±2.4 |
| KB | 9.5±5.9 | 32.5±3.0 | 22.1±1.3 |
| HepG2 | 1.7±0.6 | 28.9±2.5 | 26.1±2.1 |
| Caco-2 | -1.54±0.1 | 0.0±0.01 | -2.6±0.1 |

| | | | |
|---|---|---|---|
| *- = increase in growth | | | |

The results reported in Table 4 showed that the three peptides under investigation showed a selective cytotoxic activity. None of the compounds were cytotoxic to the CCD-18Co intestinal fibroblast line, suggesting good compatibility with healthy cells and, consequently, promising selectivity towards cancer cells. In contrast, peptides exerted a significant cytotoxic effect on several neoplastic cell lines.

In particular, with regard to HCT-8 and HCT-116 intestinal tumor lines, significant inhibitions of cell proliferation were observed, with percentages reaching about 30% for LCP2 and LCP3, significantly higher than the more contained efficacy shown by LCP1. In contrast, the Caco-2 cell line, also derived from colorectal adenocarcinoma, proved resistant to all three peptide treatments. This data suggested the possible presence of a specific mechanism of action, able to discriminate between the different molecular arrangements of HCT lines compared to that of Caco-2.

An equally relevant result emerged from the analysis of HeLa and Hep-G2 cell lines. Extending the investigation to non-intestinal tumors, the data showed that peptides exerted significant cytotoxic activity also against HeLa (cervical carcinoma) and Hep-G2 (hepatocarcinoma), with an inhibition of cell viability that was about 25% for LCP2 and LCP3. The only exception was LCP3, which showed a lower cytotoxicity against HeLa, equal to 14%.

The data obtained from the three-dimensional models (PDOs and PDTs) were consistent with what was observed in the 2D models. None of the peptides showed cytotoxic effects against PDOs, representative of the healthy intestinal mucosa, confirming the absence of toxicity towards untransformed cells. On the contrary, a significant inhibition of tumor growth was observed in PDTs, with LCP2 resulting in a reduction in tumoroid viability of 27.8%, further strengthening the evidence of a selectivity of action against neoplastic cells.

Overall, the high concordance between the results obtained in two-dimensional and three-dimensional models underlined the therapeutic potential of the three peptides, laying a solid foundation for future preclinical and clinical developments, not only in the gut but also in heterogeneous oncological contexts.

Cytotoxic activity was confirmed with Real-Time cell analyser for HCT-116 and Caco-2 lines.

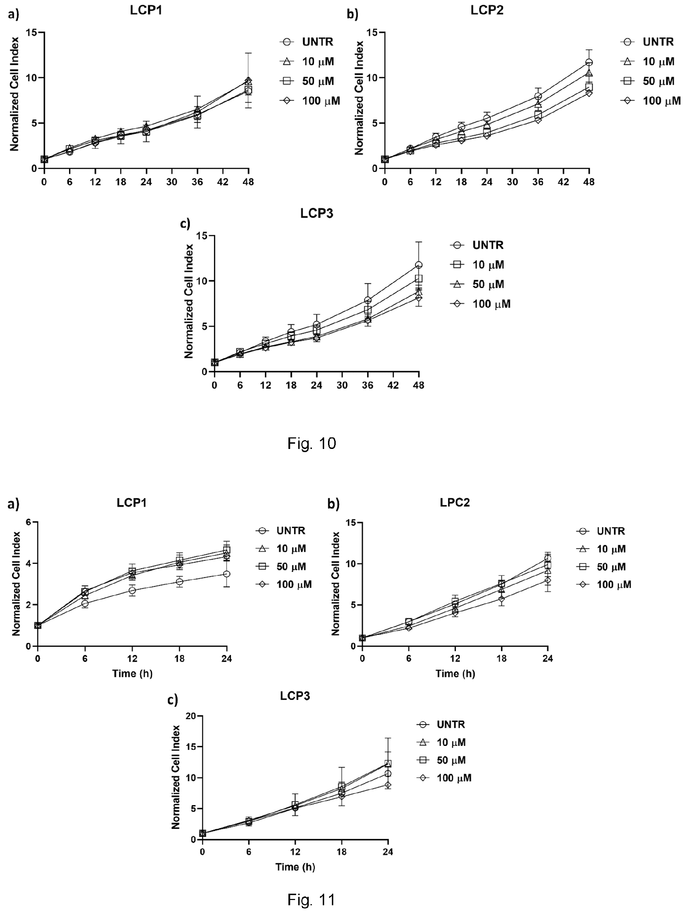

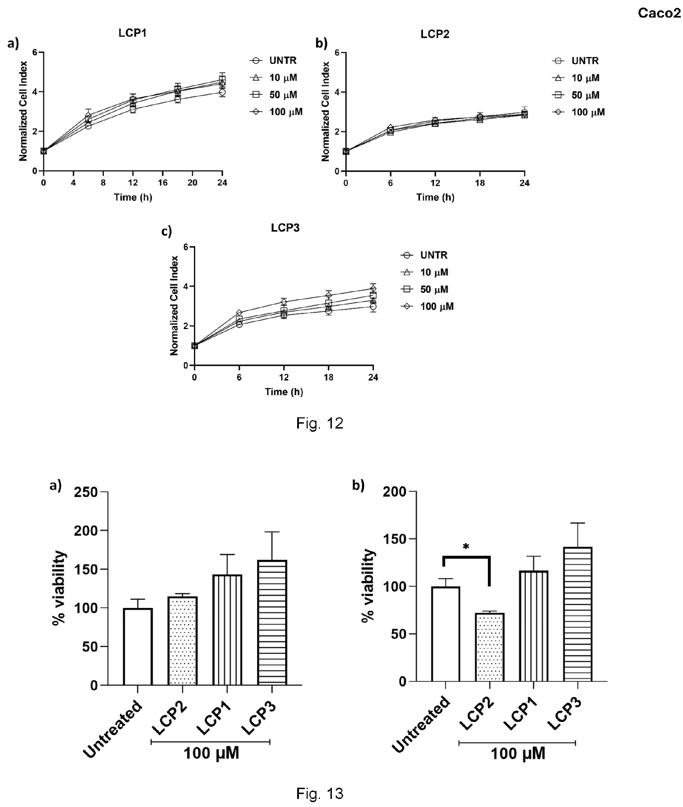

The xCELLigence technology made it possible to monitor cell proliferation in real time and without labels (label free) by measuring the electrical impedance through microelectrodes inserted in cell culture plates, allowing a dynamic evaluation of cell growth and response to treatments. In this study, the technology was used to evaluate the effects of LCP1, LCP2, and LCP3 peptides on HCT-8, HCT-116, and Caco-2 tumor cell lines over a period of 48h. In the HCT-8 cell line (Figure 10), both LCP2 and LCP3 demonstrated significant inhibitory effects on proliferation, showing a dose-dependent reduction in growth, particularly at concentrations of 50 µM and 100 µM (Fig. 10b-10c). LCP1, on the other hand, showed no detectable impact on HCT-8 proliferation at any concentration (Fig. 10a). Similarly, in the HCT-116 cell line, LCP2 was shown to be the most powerful, substantially inhibiting proliferation even at lower concentrations (Fig. 11b), while LCP3 showed moderate effects, more marked at higher doses (Fig. 11c). LCP1, consistent with previous observations, did not affect HCT-116 cell growth, reinforcing its limited efficacy in tumor inhibition (Fig. 11a). In the Caco-2 cell line, none of the peptides showed significant effects on cell growth, further confirming the resistance of this model to the compounds tested (Figure 12). Real-time data highlighted differentiated cell line responses, with LCP2 being the most powerful inhibitor of cancer cell proliferation.

### Efficacy study on organoids and tumoroids

Three-dimensional (3D) models, such as organoids and tumoroids, provide an advanced platform for evaluating therapeutic candidates under conditions that faithfully mimic the cellular architecture, microenvironment, and *in-vivo* drug response dynamics. These models bridge the gap between traditional 2D cell cultures and *in-vivo* studies, offering greater predictability of therapeutic efficacy and toxicity. In this study, organoids derived from the mucosa of the healthy colon and tumoroids from colorectal cancer tissues were treated with the peptides LCP1, LCP2, and LCP3 at a concentration of 100 µM, based on efficacy observed in other experimental settings (Figure 13). Healthy tissue-derived organoids were not affected by all three peptides, indicating a strong safety profile and the absence of off-target cytotoxicity. In contrast, tumors demonstrated significant sensitivity to LCP2, which greatly reduced viability at the tested concentration, confirming its powerful antitumor activity in a physiologically relevant 3D setting. LCP3, on the other hand, showed no reduction in viability, aligning with its inconsistent performance in other assays and suggesting limited efficacy in complex tumor models. Similarly, LCP1 had no effect on tumor viability, consistent with its lack of activity in the 2D and 3D systems.

The selective activity of LCP1, LCP2 and LCP3 peptides observed on tumor cells but not on normal cells in both 2D and 3D models, strongly suggests a mechanism that specifically targets tumor pathways. Indeed, this selectivity was supported by the absence of cytotoxic or anti proliferative effects on normal fibroblasts (CCD-18Co) and organoids derived from healthy mucosa, indicating that peptides act through a tumor-specific mechanism.

Advantageously, the side effects of peptide compounds according to the invention, administered as such or in the form of pharmaceutical compositions, in neutral form or as derived salts, are significantly lower than those generated by anticancer analogues such as 5-fluoroacyl or cisplatin, or appear to be totally absent. In particular, the undesirable effects such as psychological damage, physical regression and economic outlay related to a therapy with traditional anticancer drugs are drastically contained, reduced or even cancelled.

### METHOD

Below is a possible synthetic route for obtaining peptide compounds according to the invention, described in the essentiality of its phases (Scheme 1). The technical measures necessary for the implementation of the following method are absolutely within the reach of the average technician in the field.

Compounds of formula **(II)** according to the present invention may be obtained by the method described below or by slight modifications of this method which are in any case within the reach of the expert in the field.

Any expert in the field, combining the technical teachings of this synthetic method with his own general knowledge (common general knowledge), will be perfectly able to synthesize even compounds of general formula **(I).**

### Synthesis method

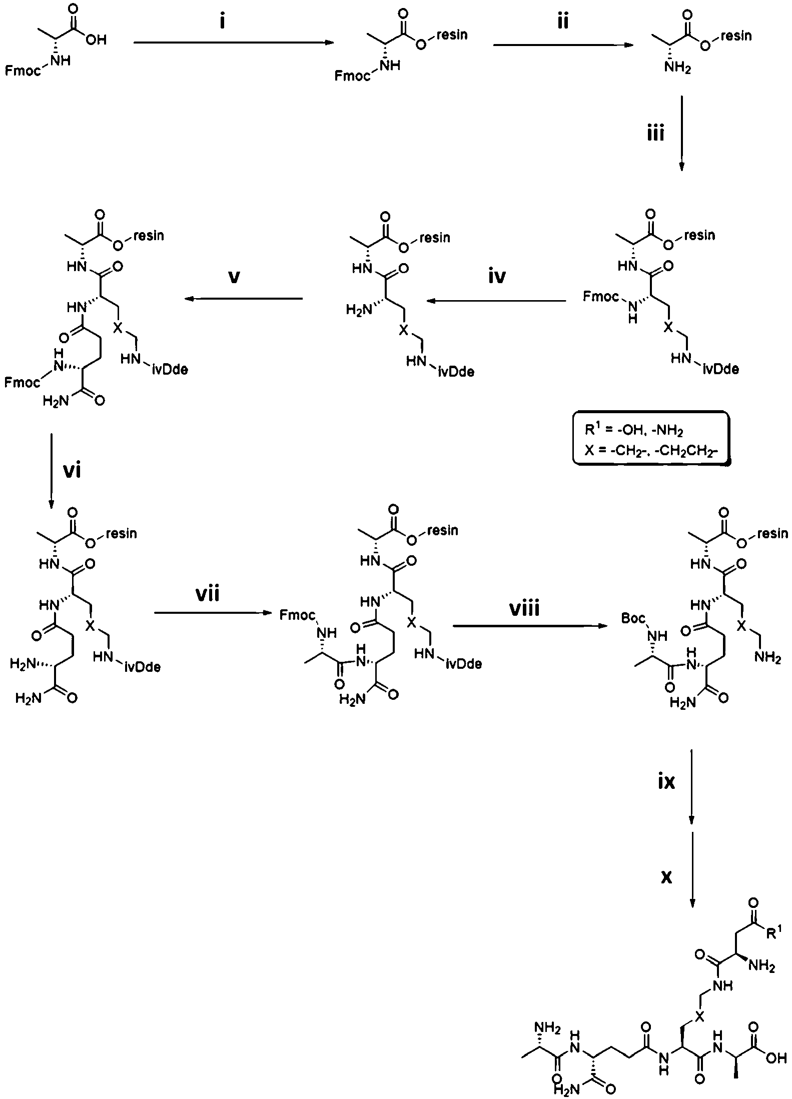

**Scheme** 1 Synthesis of formula **(II)** compounds. Reaction conditions and reagents: *i)* 2-chlorotrityl chloride resin, N,N-diisopropylethylamine (DIPEA), dichloromethane (DCM); *ii)* 20% piperidine in dimethylformamide (DMF); *iii)* Fmoc-L-Orn(ivDde)-OH or Fmoc-L-Lys(ivDde)-OH, Ethyl cyanohydroxyiminoacetate (Oxyma), N,N'-Diisopropylcarbodiimide (DIC), DMF; *iv)* 20% piperidine in dimethylformamide (DMF); *v)* Fmoc-D-iGln-OH, Oxyma, DIC, DMF; *vi)* 20% piperidine in dimethylformamide (DMF); ***vii)*** Fmoc-D-Ala-OH, Oxyma, DIC, DMF; ***viii)*** There are 3 steps: 1) 20% piperidine in dimethylformamide (DMF); 2) Di-tert-butyl dicarbonate (Boc₂O), DIPEA; 3) 2% hydrazine in DMF; ***ix)*** Fmoc-D-Asn-OH or Fmoc-D-Asp(OtBu)-OH, Oxyma, DIC, DMF; ***x***) Trifluoroacetic acid (TFA) / triisopropylsilane (TIPS), phenol, water.

The peptide sequences were synthesized using the standard approach of Fmoc solid-phase peptide synthesis, known to an expert in the field. All Fmoc-(fluorenyl methoxycarbonyl) amino acids present in the sequence contained classical acid-labile protective groups for side chains, with the exception of Lis (Lysine) and Orn (Ornithine), which were protected with the -(ivDde) group. A 2-chlorotrityl chloride resin (Novabiochem^{®}, load capacity 1.47 mmol/g, 0.3 g) was used as a solid support.

For resin loading, 2 equivalents of Fmoc-protected amino acid Ala (Alanine) and 8 equivalents of DIPEA were dissolved in anhydrous DCM (3 mL) and the reaction mixture was added to the resin. The mixture was shaken on a tube roller mixer overnight at room temperature. The resin was then washed three times with DCM (20 mL) and DMF (20 mL), followed by an end-capping treatment (a procedure known to an expert in the branch to ensure that the reactive groups of the resin have fully reacted and are not available for further amide bonds with the subsequent amino acid) with MeOH. Fmoc deprotection was performed by treating the resin twice for 15 minutes with a 20% piperidine/DMF solution (10 mL).

A standard protocol was used for the elongation of the peptide chain: at each step, the resin was reacted with 4 equivalents of Fmoc-protected amino acid, 4 equivalents of Oxyma and 4 equivalents of DIC in anhydrous DMF (3 mL). The reaction mixture was stirred on a roller mixer for 18 hours at room temperature. Fmoc deprotections were performed with 20% piperidine/DMF (10 mL).

At the end of the sequence, the last amino acid was deprotected from the Fmoc group and protected by reaction with 20 Boc₂O equivalents and 20 DIPEA equivalents in an anhydrous DMF solution (3 mL), then added to the resin and stirred for 30 minutes. After removal of the ivDde group from the Lis or Orn in the presence of a 2% hydrazine solution in DMF (10 mL), the amino acid was specifically branched with Asn (Asparagine) or Asp (Aspartic Acid), coupled using the same Oxyma/DIC protocol already described.

After the coupling process, the peptides were removed from the resin. For detachment from the solid phase, the resin was treated for 1 hour with a solution of TFA/TIPS (triisopropylsilane)/phenol/water (97/1/1/1, 10 mL). The solutions were then concentrated, and the raw products were isolated by precipitation in cold diethyl ether. The precipitates were collected by centrifugation and dried under vacuum. The raw products were analyzed using RP-HPLC and mass spectrometry.

### USE

The subject of this invention are the compounds of formula **(I)** and **(II)** according to the invention for use as a medicament, preferably for use as antitumor drugs.

In particular, the peptide compounds of formula **(I)** and **(II)** of the invention are suitable for use as anticancer drugs in the treatment of:
- Colon carcinoma;
- Basal cell carcinoma;
- Cervical carcinoma.

### EXAMPLES

The contents of this paragraph, the examples given and the preparation method are to be understood as illustrative descriptions only, which are in no way intended to limit the scope of the present invention.

The compounds of formula **(I)** and **(II)** according to the present invention can be made either by slavishly following the methods described below or by slight modifications of the same.

These slight modifications, to be considered within the reach of an average technician in the field, may concern reaction times, order of addition of ingredients, temperature, and any other variable known to the expert in the field, without altering the result or extending the scope of the invention itself.

### Materials

All chemicals were purchased from Merk, Sigma Aldrich and Acros Organics and are reagent grade or higher. The solvents were purchased from Fisher Scientific, Sigma Aldrich and VWR. The samples were dried in a vacuum furnace (Gallenkamp, with a maximum temperature of ≤ 250°C) connected to a vacuum pump (BOC-Edwards). Mass spectrometry was performed on a Thermofisher LCQ DECA XP ION Trap Mass Spectrometer. HPLC analyses were conducted using an Agilent 1100 Series HPLC system with a quadrupole pump, vacuum degasser, variable wavelength detector (set at 254 or 281 nm), and a mobile phase consisting of a water/acetonitrile gradient. The HPLC column used was an Agilent Eclipse XDB-C18 (4.6 mm × 150 mm) with a guard column.

### Cells

Human colorectal cells HCT116 (CCL-247^{™}), HCT8 (CCL-244^{™}), and Caco-2 (HTB-37^{™}), as well as KB papilloma cells (CRL-3596^{™}), Hep G2 liver carcinoma cells (HB-8065^{™}), H1HeLa cervical carcinoma cells (CRL-1958^{™}), and CCD-18Co unprocessed human colon fibroblasts (CRL-1459^{™}) were purchased from ATCC Company (Manassas, Virginia, United States).

HCT116 and HCT8 cells were suspended in RPMI 1640 (Gibco, Cat. No. 21870076). The Caco-2 cells were retained in Dulbecco's Modified Eagle Medium (DMEM) (1-26F03-I, BioConcept Ltd., Allschwil, Switzerland). The KBs, Hep G2 and CCD-18Co cells were maintained in Eagle's Minimum Essential Terrain (EMEM) (30-2003^{™}, ATCC Company). Finally, H1HeLa cells were retained in Leibovitz's L-15 Medium (30-2008^{™}, ATCC Company).

All culture media contained 10% fetal bovine serum (FBS, Gibco, cat. no. 10082147), 100 U/mL penicillin and 100 µg/mL streptomycin (Gibco, cat. no. 15070063). At 80% confluence, cells were passed with a trypsin-EDTA solution (0.05% trypsin and 0.02% EDTA, Gibco, cat. no. 25300054).

### In silico analysis.

The crystal structure of the MDM2-p53 protein (PDB ID: 1YCR) was collected from the Protein Data Bank (PDB, http://www.rcsb.org/pdb) and optimized with the YASARA software. The structure of Hexokinase 2 (5HG1) can be consulted in https://www.rcsb.orq/structure/5HG1 and https://www.rcsb.org.

Molecular docking experiments of the compounds with the selected protein were performed with AutoDock Vina supplied in YASARA. The 2D chemical structure of peptides was constructed with MarvinSketch (https://chemaxon.com/products/marvin) to obtain a 3D molecule from a 2D structure and saved in the PDB format. A docking was performed using a cell with an extension that comprises all atoms extending 5 A° from the outside of the p53 structure. The screening uses the YASARA structure software with the runscreening.mcr macro dock set with the VINA method, runs = 100 and force field AMBER14. All parameters have been entered with the default settings, as previously reported. Docking results were analyzed with the BIOVIADiscovery Study.

### Evaluation of the cytotoxicity or anti proliferative effects of the invention's peptides.

To evaluate the anti proliferative and cytotoxic effects of the peptide on tumor cell lines, the assay of 3-(4,5-dimethylthiazol-2-yl)-2,5-diphenyl tetrazolium bromide (MTT), known to an expert in the field, was conducted. Cell viability was determined by colorimetric reduction of MTT, catalysed by mitochondrial succinate dehydrogenase. Prior to exposure, 100 µL of cell line suspension was seeded into a 96-well microplate at a density of 1.5x10⁴ cells/well and incubated for 18-20 hours. After incubation, cells were treated with LCP1, LCP2, and LCP3 at 10, 50, and 100 µM, with or without 10% FBS respectively, to assess antiproliferative or cytotoxic activity. Then, the cells were incubated at 37°C overnight in 5% CO₂. Next, 100 µL of a tetrazolium salt solution (0.5 mg/mL) were added to each well and were incubated at 37°C in 5% CO₂ for 3 hours. After incubation, tetrazolium salts were solubilized with 100 µL of DMSO, and optical density (OD) was measured at a wavelength of 570 nm using a microplate spectrophotometer. Four tests were performed for each sample, and the results were expressed as a mean value ± standard deviation (SD).

### Real-time monitoring of cell proliferation

The xCELLigence experiments were conducted following the methodology described by Fuochi et al. (10.3389/fmolb.2023.1082526).

The Dual Plate (DP) Real-Time Cell Analyzer (RTCA) instrument was used in accordance with the manufacturer's guidelines. The ideal cell seeding density was determined by cell titration and growth assays. Once the optimal number of cells (3.0×10³ cells/well) was established, the cells were automatically monitored every 30 minutes for 24 hours. The optimal seeding density was determined in preliminary experiments to ensure a significant cell index (CI) value and consistent cell growth throughout the experiment. For this type of analysis, two cell lines were chosen that are more representative of the mechanism of action of the molecules in question. The two cell lines selected were HCT-116 and Caco-2.

### Establishment and maintenance of patient-derived organoid cultures (PDOs)

The collection of samples and experiments have been approved by the Ethics Commission 2 - Catania (I) prot.601/C.E.

Biopsies of healthy mucosa and tumor were obtained from patients with colon adenocarcinoma at the "Fondazione Mediterranea G.B. Morgagni, Catania, Italy". The studies were conducted in accordance with the Declaration of Helsinki, and all participants signed the informed consent. Treatment of biopsies and isolation of crypts were performed following the methodological guidelines of Sato *et al.* 2011 [2]

To allow the organoids to develop correctly *in vitro,* mechanical and nutritional support was activated by inserting the crypts into Matrigel domes (Matrigel Growth Factor Reduced Basement Membrane Matrix, Corning). Once the organoid cultures were obtained, they were subjected to passes every 7 days and the medium was replaced every 48 hours. Culture maintenance was performed using the IntestiCultTM Organoid Growth Medium kit (StemCellTM technologies) supplemented with 1% penicillin/streptomycin (100× stock, Gibco).

### Treatment of PDOs with peptides and cytotoxicity assay - 3D

Intestinal organoids from the healthy and cancer mucosa were seeded and incubated for 48 hours. Then, the organoids in each well were counted (40-80 organoids per well) to perform the final normalization of the viability data. The peptides were appropriately dissolved to obtain final concentrations of 10, 50 and 100 µM in the growing medium. Both healthy and tumor organoids were treated with the aforementioned peptides for 24 hours. Then, the working solution of 3-(4,5-dimethylthiazol-2-yl)-2,5-diphenyltetrazoliumbromide (MTT, Merck) was prepared at a concentration of 5 mg/mL by dissolving tetrazolium salts in Dulbecco's 1X phosphate saline (DPBS). An adequate volume of salt solution was added to each well to achieve a final concentration of 0.5 mg/mL. The organoids were incubated for 30 minutes to 1 hour and the formation of formazan crystals was monitored every 20 minutes.

After incubation, the supernatant was gently removed, a volume of 2% sodium dodecyl sulphate (SDS 2%) was added, and it was allowed to incubate for 20-30 minutes so that the domes dissolved.

Then, after pipetting gently to dissolve any solid residue, 200 µL of Dimethyl Sulfoxide (DMSO) were added to each well and the plate was incubated for 10-15 minutes. Finally, the reading was taken by setting the instrument to the wavelength of 562 nm.

Below are some embodiments of the present invention.

### EXAMPLE 1 - LCP1 (compound of known art): N₂-(d-asparaginyl)-N₆-((R)-5-amino-4-((S)-2-aminopropanamido)-5-oxopentanoyl)-I-lysyl-d-alanine.

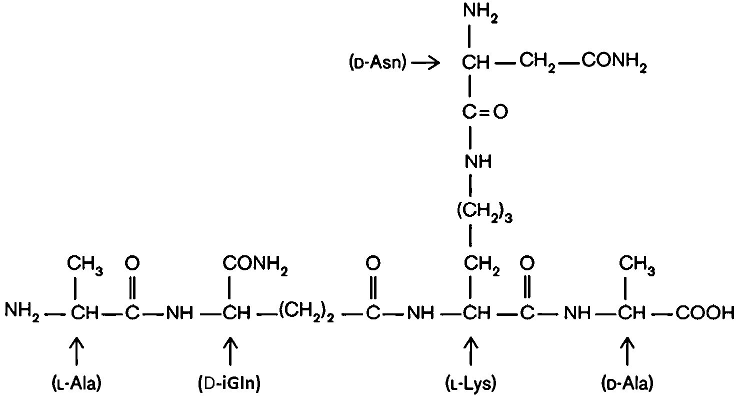

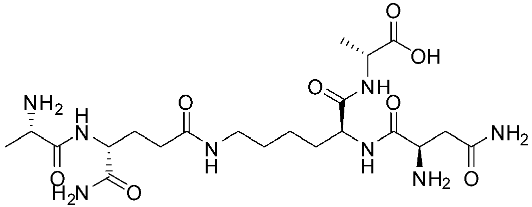

**Synthesis.** A 2-chlorotrityl chloride resin (Novabiochem^{®}, load capacity 1.47 mmol/g, 0.3 g) was used as a solid support. For resin loading, 2 Fmoc-protected Ala amino acid equivalents and 8 equivalents of DIPEA were dissolved in anhydrous DCM (3 mL) and the reaction mixture was added to the resin. The mixture was shaken on a tube roller mixer overnight at room temperature. The resin was then washed three times with DCM (20 mL) and DMF (20 mL), followed by an end-capping treatment with MeOH.

For FMOC deprotection, the resin was treated twice for 15 minutes with a 20% piperidine/DMF solution (10 mL). Subsequently, a standard protocol for peptide elongation was used. At each pass, the resin was reacted with 4 Fmoc-protected amino acid equivalents, 4 Oxyma equivalents and 4 DIC equivalents (N, N'-Diisopropylcarbodiimide) in anhydrous DMF (3 mL). The reaction mixture was stirred on a roller mixer for 18 hours at room temperature. Fmoc deprotections were performed with 20% piperidine/DMF (10 mL).

At the end of the sequence, the last amino acid was deprotected from the Fmoc group and protected by reaction with 20 ditert-butyl dicarbonate equivalents and 20 DIPEA equivalents in an anhydrous DMF solution (3 mL), then added to the resin and stirred for 30 minutes. After removal of the ivDde group from the Lys in the presence of a 2% hydrazine solution in DMF (10 mL), the amino acid was specifically branched with Asn, coupled using the same Oxyma/DIC protocol already described.

After coupling, the peptide was removed from the resin. For detachment from the solid phase, the resin was treated for 1 hour with a solution of TFA/TIPS (triisopropylsilane)/phenol/water (97/1/1/1, 10 mL). The solution was then concentrated, and the crude product was isolated by precipitation in cold diethyl ether. The precipitates were collected by centrifugation and dried under vacuum. The raw products were analyzed by RP-HPLC and mass spectrometry.

**C₂₁H₃₉N₈O₈⁺ [M+H]⁺** (Mass ESI+): CALC (531.29) vs FOUND (531.34)
Mass spectrometry analysis (Mass ESI+) provided confirmation of molecular identity thanks to the exact correspondence between the observed mass and the calculated molecular weight: m/z: 531.29

**HPLC PURITY:** 99%

### EXAMPLE 2 - LCP2: (R)-3-amino-4-(((S)-6-((R)-5-amino-4-((S)-2-aminopropanamido)-5-oxopentanamido)-1-(((R)-1-carboxyethyl) amino)-1-oxohexan-2-yl)amino)-4-oxobutanoic acid

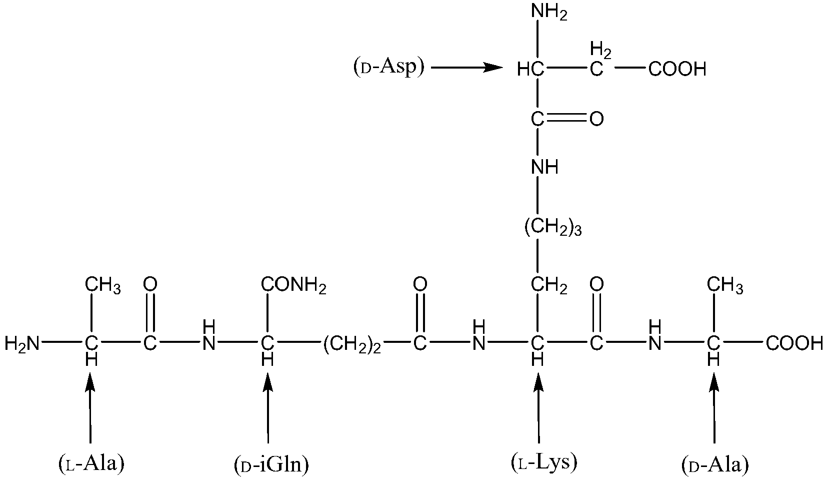

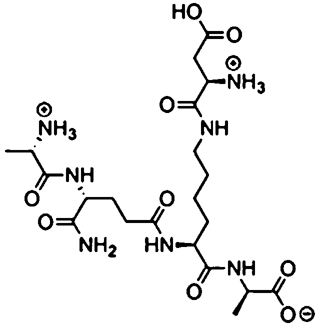

**Synthesis.** A 2-chlorotrityl chloride resin (Novabiochem^{®}, load capacity 1.47 mmol/g, 0.3 g) was used as a solid support. For resin loading, 2 Fmoc-protected Ala amino acid equivalents and 8 equivalents of DIPEA were dissolved in anhydrous DCM (3 mL) and the reaction mixture was added to the resin. The mixture was shaken on a tube roller mixer overnight at room temperature. The resin was then washed three times with DCM (20 mL) and DMF (20 mL), followed by an end-capping treatment with MeOH.

For FMOC deprotection, the resin was treated twice for 15 minutes with a 20% piperidine/DMF solution (10 mL). Subsequently, a standard protocol for peptide elongation was used. At each pass, the resin was reacted with 4 Fmoc-protected amino acid equivalents, 4 Oxyma equivalents and 4 DIC equivalents (N, N'-Diisopropylcarbodiimide) in anhydrous DMF (3 mL). The reaction mixture was stirred on a roller mixer for 18 hours at room temperature. Fmoc deprotections were performed with 20% piperidine/DMF (10 mL).

At the end of the sequence, the last amino acid was deprotected from the Fmoc group and protected by reaction with 20 ditert-butyl dicarbonate equivalents and 20 DIPEA equivalents in an anhydrous DMF solution (3 mL), then added to the resin and stirred for 30 minutes. After removal of the ivDde group from the Lys in the presence of a 2% hydrazine solution in DMF (10 mL), the amino acid was specifically branched with Asp, coupled using the same Oxyma/DIC protocol already described.

After coupling, the peptide was removed from the resin. For detachment from the solid phase, the resin was treated for 1 hour with a solution of TFA/TIPS (triisopropylsilane)/phenol/water (97/1/1/1, 10 mL). The solution was then concentrated, and the crude product was isolated by precipitation in cold diethyl ether. The precipitates were collected by centrifugation and dried under vacuum. The raw products were analyzed by RP-HPLC and mass spectrometry.

**C₂₁H₃₈N₇O₉⁺ [M+H]⁺** (Mass ESI+): CALC (532.27) vs FOUND (532.21)
Mass spectrometry analysis (Mass ESI+) provided confirmation of molecular identity thanks to the exact correspondence between the observed mass and the calculated molecular weight: m/z: 532.27

**HPLC PURITY:** 99%.

### EXAMPLE 3 - LCP3: ((S)-2-((R)-5-amino-4-((S)-2-aminopropanamido)-5-oxopentanamido)-5-((R)-2,4-diamino-4-oxobutanamido) pentanoyl)-D-alanine

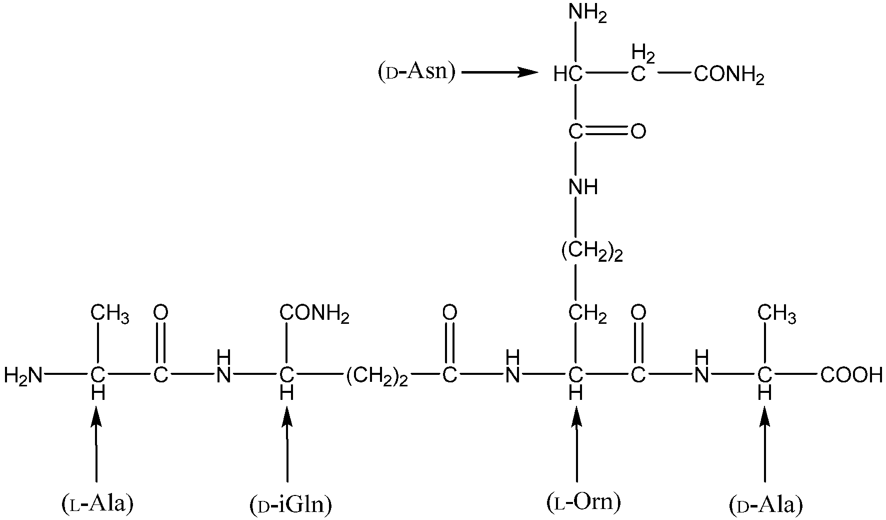

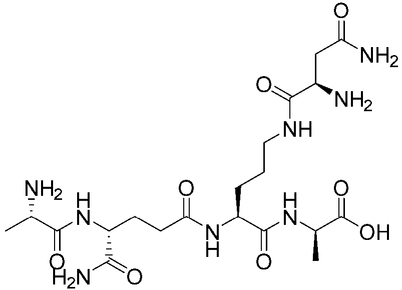

**Sythesis.** A 2-chlorotrityl chloride resin (Novabiochem^{®}, load capacity 1.47 mmol/g, 0.3 g) was used as a solid support. For resin loading, 2 Fmoc-protected Ala amino acid equivalents and 8 equivalents of DIPEA were dissolved in anhydrous DCM (3 mL) and the reaction mixture was added to the resin. The mixture was shaken on a tube roller mixer overnight at room temperature. The resin was then washed three times with DCM (20 mL) and DMF (20 mL), followed by an end-capping treatment with MeOH.

For FMOC deprotection, the resin was treated twice for 15 minutes with a 20% piperidine/DMF solution (10 mL). Subsequently, a standard protocol for peptide elongation was used. At each pass, the resin was reacted with 4 Fmoc-protected amino acid equivalents, 4 Oxyma equivalents and 4 DIC equivalents (N, N'-Diisopropylcarbodiimide) in anhydrous DMF (3 mL). The reaction mixture was stirred on a roller mixer for 18 hours at room temperature. Fmoc deprotections were performed with 20% piperidine/DMF (10 mL). At the end of the sequence, the last amino acid was deprotected from the Fmoc group and protected by reaction with 20 ditert-butyl dicarbonate equivalents and 20 DIPEA equivalents in an anhydrous DMF solution (3 mL), then added to the resin and stirred for 30 minutes. After removal of the ivDde group from the Orn in the presence of a 2% hydrazine solution in DMF (10 mL), the amino acid was specifically branched with Asn, coupled using the same Oxyma/DIC protocol already described. After coupling, the peptide was removed from the resin. For detachment from the solid phase, the resin was treated for 1 hour with a solution of TFA/TIPS (triisopropylsilane)/phenol/water (97/1/1/1, 10 mL). The solution was then concentrated, and the crude product was isolated by precipitation in cold diethyl ether. The precipitates were collected by centrifugation and dried under vacuum. The raw products were analyzed by RP-HPLC and mass spectrometry.

**C₂₀H₃₇N₈O₈* [M+H]⁺** (Mass ESI+): CALC (517.27) vs FOUND (517.50)
Mass spectrometry analysis (Mass ESI+) provided confirmation of molecular identity thanks to the exact correspondence between the observed mass and the calculated molecular weight: m/z: 517.27

**HPLC PURITY:** 99%

### Results discussion

Computational molecular docking analysis was a central element in the hit-to-lead (H2L) optimization phase of LCP1-derived peptides, allowing to correlate specific structural changes with predictable changes in affinity towards molecular targets involved in tumor survival.

The results obtained suggest that the developed compounds do not act exclusively as non-specific cytotoxic agents but can modulate biological pathways associated with neoplastic proliferation and metabolism. In particular, the simulations showed a double mechanistic potential: on the one hand, interference with the MDM2/p53 axis, and on the other hand, a marked affinity for hexokinase-2 (HK2/E2), a key enzyme of tumor glycolytic metabolism.

As far as MDM2 is concerned, the most advanced peptides showed a progressive improvement in binding parameters compared to the initial LCP1 lead. Gln/Glu derivatives, and in particular LCP3bGln, reached ΔG_{leg} values up to -8.0 kcal/mol, with predicted Kᵢ equal to about 1 µM, significantly better than LCP1 (-5.7 kcal/mol; Kᵢ 65 µM). This increase in affinity suggests that the changes introduced have favored greater steric and electrostatic complementarity with the functional groove of MDM2.

Of particular relevance is the fact that peptides repeatedly interact with residues known to be involved in the recognition of the transactivating region of p53, comprising Leu54, Phe55, Ile61, Met62, Tyr67, His73, Phe91, Ile99 and Tyr100. These residues constitute the hydrophobic core of the MDM2 pocket and represent functionally critical sites for the maintenance of the MDM2/p53 interaction. The repeated involvement of these amino acids suggests that the peptides do not occupy peripheral or non-specific regions of the protein but insert themselves into the biologically relevant site responsible for the interaction with p53.

The acquisition of additional interactions with residues such as Lys51, Gln71, GIn72 and Lys94 appears particularly interesting from a conformational point of view, since it could contribute to the stabilization of the peptide-protein complex through a more extensive network of hydrogen bonds and polar interactions. This is consistent with the progressive improvement in ΔG_{leg} values observed in derivatives containing glutamine or glutamate. In biological terms, these results support the hypothesis that these compounds may reduce the ability of MDM2 to sequester and degrade p53, indirectly promoting the restoration of apoptotic and antiproliferative pathways in cancer cells.

The data relating to HK2/E2 appear even more relevant. All the optimized compounds showed higher affinity values than those observed for MDM2, with ΔG_{leg} ranging from -9.3 to -10.5 kcal/mol and Kᵢ predicted in the sub micromolar order, up to values in the order of tens of nano molars. In particular, LCP3GIn achieved a ΔG_{leg} of -10.5 kcal/mol with Kᵢ equal to 0.02 µM, while LCP2GIn and LCP3Glu showed Kᵢ values equal to about 0.04 µM.

From a pharmacodynamic point of view, these values are indicative of a possible stable interaction with the functional site of the enzyme. HK2 represents one of the main regulators of tumor glycolytic metabolism and is frequently overexpressed in tumors with high proliferative activity. Its association with the outer mitochondrial membrane allows not only an efficient use of glucose, but also an important anti-apoptotic protection. As a result, HK2 inhibition can simultaneously lead to metabolic stress, reduced ATP production, mitochondrial destabilization and activation of apoptotic mechanisms.

The observed interactions involve numerous functionally relevant residues of the catalytic pocket and adjacent regions, comprising Lys621, Thr620, Gly681, Ser682, Asn683, Glu708, Leu734, Asn735, Lys738, GIn739, Glu742, Lys743, Met748 and Tyr749. The recurrent involvement of these residues suggests a structurally conserved binding mode compatible with a possible interference on enzyme activity.

It is particularly important to note that the computational improvement of affinity was consistent with the available experimental biological data. Peptides showing favourable docking parameters, in particular LCP2 and LCP3, were also those that produced the most marked anti proliferative effects in the HCT-8 and HCT-116 lines, with reduced cell viability, dose-dependent effect and confirmation by xCELLigence system. In parallel, the absence of significant toxicity in CCD-18Co fibroblasts and healthy intestinal organoids suggests that the observed effect is not due to indiscriminate cytotoxicity, but rather to a specific biological vulnerability of cancer cells.

The resistance observed in the Caco-2 line is also biologically consistent with this interpretation. Caco-2 cells have a highly differentiated phenotype and different metabolic characteristics than HCT lines, comprising a possible lower dependence on glycolysis and HK2. This data suggests that peptides may preferentially target cells metabolically dependent on the tumor glycolytic axis, further strengthening the hypothesis of a selective and not generically toxic mechanism.

Overall, docking data should not be interpreted as definitive evidence of pharmacological activity, but as preliminary mechanistic evidence supporting observed activity. However, the concordance between SAR optimization, progressive increase in computational affinity, involvement of functionally relevant residues and experimental confirmation in 2D and 3D models gives the developed compounds a promising profile as innovative anticancer candidates.

### Bibliography

[1] Antitumoral activity of a cytotoxic peptide of Lactobacillus casei peptidoglycan and its interaction with mitochondrial-bound hexokinase; Giuseppe A Fichera 1, Marco Fichera, Giuseppe Milone; DOI: 10.1097/CAD.0000000000000367
[2] Single Lgr5 stem cells build crypt-villus structures *in vitro* without a mesenchymal niche; Sato T, Vries RG, Snippert HJ, van de Wetering M, Barker N, Stange DE, van Es JH, Abo A, Kujala P, Peters PJ, Clevers H. DOI: 10.1038/nature07935.

## Claims

1. A compound represented by formula **(I):** 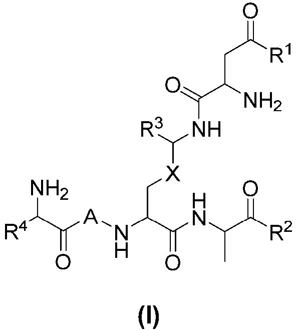 wherein
**X** = -CH₂-, -CH₂CH₂-;
R¹ = -OH, -NH₂;
**R²** = -OH, 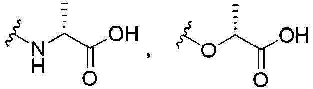
**R³** = -H, -COOH;
**R⁴** = -H, -CH₃; 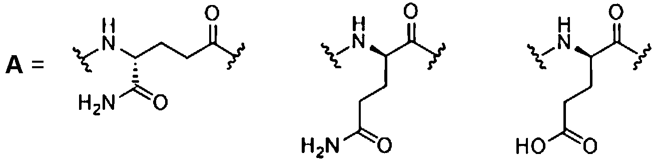
or a salt, zwitterion, stereoisomer, solvate, or a pharmaceutically acceptable combination thereof, and
excluding LCP1 compound 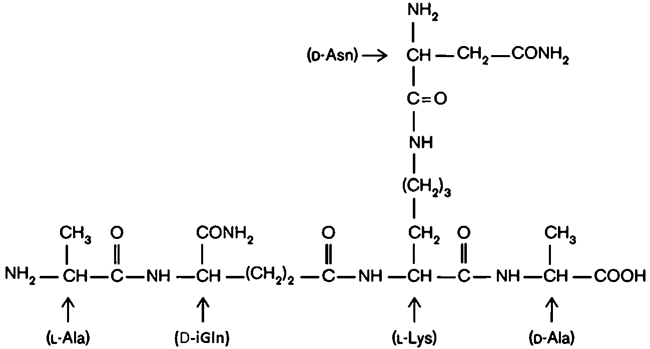
regardless of its protonation state and its neutral, zwitterionic, saline, solvated, or stereoisomeric form.

2. The compound according to the preceding claim represented by formula **(II):** 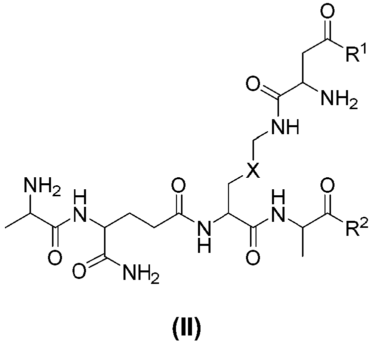 wherein
**X** = -CH₂-, -CH₂CH₂-;
**R¹** = -OH, -NH₂;
R² = -OH, 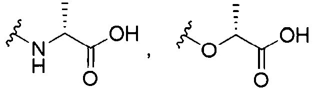
or its salt, its zwitterion, its stereoisomer, its solvate or a pharmaceutically acceptable combination thereof.

3. The compound according to anyone of the preceding claims chosen from: LCP2, LCP3, LCP1Ala, LCP2Lat, LCP3Ala, LCP1Gln, LCP2GIn, LCP3GIn, LCP1bGln, LCP2bGln, LCP3bGln, LCP1Glu, LCP2Glu, LCP3Glu, LCP1bGlu, LCP2bGlu, LCP3bGlu, and mixtures thereof, wherein:
LCP1Ala is identified by the sequence SEQ ID NO: 1 = AXKAA
LCP2 is identified by the sequence SEQ ID NO: 2 = AXKA
LCP3 is identified by the sequence SEQ ID NO: 3 = AXXA
LCP2Lat is identified by the sequence SEQ ID NO: 4 = AXKAX
LCP3Ala is identified by the sequence SEQ ID NO: 5 = AXXAA
LCP1Gln is identified by the sequence SEQ ID NO: 6 = AQKNA
LCP2GIn is identified by the sequence SEQ ID NO: 7 = AQKDA
LCP3GIn is identified by the sequence SEQ ID NO: 8 = AQXA
LCP1bGln is identified by the sequence SEQ ID NO: 9 = AQKNAA
LCP2bGln is identified by the sequence SEQ ID NO: 10 = AQKDAX
LCP3bGln is identified by the sequence SEQ ID NO: 11 = AQXAA
LCP1Glu is identified by the sequence SEQ ID NO: 12 = AQKNA
LCP2Glu is identified by the sequence SEQ ID NO: 13 = AEKDA
LCP3Glu is identified by the sequence SEQ ID NO: 14 = AEXA
LCP1bGlu is identified by the sequence SEQ ID NO: 15 = AEKNAA
LCP2bGlu is identified by the sequence SEQ ID NO: 16 = AEKDAX
LCP3bGlu is identified by the sequence SEQ ID NO: 17 = AEXAA.

4. The compound according to anyone of the preceding claims, wherein A comprises or is a portion derived from glutamine or glutamate.

5. The compound according to anyone of the preceding claims, chosen from: LCP1Gln, LCP2GIn, LCP3GIn, LCP1bGln, LCP2bGln, LCP3bGln, LCP1Glu, LCP2Glu, LCP3Glu, LCP1bGlu, LCP2bGlu, LCP3bGlu and mixtures thereof, wherein:
LCP1Gln is identified by the sequence SEQ ID NO: 6 = AQKNA
LCP2GIn is identified by the sequence SEQ ID NO: 7 = AQKDA
LCP3GIn is identified by the sequence SEQ ID NO: 8 = AQXA
LCP1bGln is identified by the sequence SEQ ID NO: 9 = AQKNAA
LCP2bGln is identified by the sequence SEQ ID NO: 10 = AQKDAX
LCP3bGln is identified by the sequence SEQ ID NO: 11 = AQXAA
LCP1Glu is identified by the sequence SEQ ID NO: 12 = AQKNA
LCP2Glu is identified by the sequence SEQ ID NO: 13 = AEKDA
LCP3Glu is identified by the sequence SEQ ID NO: 14 = AEXA
LCP1bGlu is identified by the sequence SEQ ID NO: 15 = AEKNAA
LCP2bGlu is identified by the sequence SEQ ID NO: 16 = AEKDAX
LCP3bGlu is identified by the sequence SEQ ID NO: 17 = AEXAA
and mixtures thereof.

6. The compound according to anyone of the preceding claims, chosen from: LCP1Gln, LCP2GIn, LCP3GIn, LCP1bGln, LCP2bGln, LCP3bGln and mixtures thereof, wherein:
LCP1Gln is identified by the sequence SEQ ID NO: 6 = AQKNA
LCP2GIn is identified by the sequence SEQ ID NO: 7 = AQKDA
LCP3GIn is identified by the sequence SEQ ID NO: 8 = AQXA
LCP1bGln is identified by the sequence SEQ ID NO: 9 = AQKNAA
LCP2bGln is identified by the sequence SEQ ID NO: 10 = AQKDAX
LCP3bGln is identified by the sequence SEQ ID NO: 11 = AQXAA
and mixtures thereof.

7. The compound according to anyone of the preceding claims, chosen from: LCP1Glu, LCP2Glu, LCP3Glu, LCP1bGlu, LCP2bGlu, LCP3bGlu and mixtures thereof, wherein:
LCP1Glu is identified by the sequence SEQ ID NO: 12 = AQKNA
LCP2Glu is identified by the sequence SEQ ID NO: 13 = AEKDA
LCP3Glu is identified by the sequence SEQ ID NO: 14 = AEXA
LCP1bGlu is identified by the sequence SEQ ID NO: 15 = AEKNAA
LCP2bGlu is identified by the sequence SEQ ID NO: 16 = AEKDAX
LCP3bGlu is identified by the sequence SEQ ID NO: 17 = AEXAA
and mixtures thereof.

8. The compound according to anyone of the preceding claims, chosen from: LCP3bGln, LCP3GIn, LCP2GIn, LCP3Glu, LCP2Glu and mixtures thereof, wherein:
LCP3GIn is identified by the sequence SEQ ID NO: 8 = AQXA
LCP2GIn is identified by the sequence SEQ ID NO: 7 = AQKDA
LCP3Glu is identified by the sequence SEQ ID NO: 14 = AEXA
LCP2Glu is identified by the sequence SEQ ID NO: 13 = AEKDA
LCP3bGln is identified by the sequence SEQ ID NO: 11 = AQXAA
and mixtures thereof.

9. The compound according to anyone of the 1-3 claims, chosen from: LCP2, LCP3, LCP1Ala, LCP2Lat, LCP3Ala and mixtures thereof, wherein:
LCP1Ala is identified by the sequence SEQ ID NO: 1 = AXKAA
LCP2 is identified by the sequence SEQ ID NO: 2 = AXKA
LCP3 is identified by the sequence SEQ ID NO: 3 = AXXA
LCP2Lat is identified by the sequence SEQ ID NO: 4 = AXKAX
LCP3Ala is identified by the sequence SEQ ID NO: 5 = AXXAA
and mixtures thereof.

10. A pharmaceutical composition comprising at least one compound according to anyone of claims 1-9 and at least one pharmaceutically acceptable excipient.

11. The pharmaceutical composition according to claim 10 comprising at least one component chosen from:
• at least one pharmaceutical vehicle;
• at least one solubilizing agent;
• at least one stabilizer;
• at least one thickener;
• at least one gelling agent;
and combinations thereof;
wherein said at least one stabilizer, is preferably chosen from:
• antioxidants, preferably ascorbic acid, sodium metabisulfite and parabens;
• chelating agents, preferably EDTA;
• protein stabilizers, preferably lecithin or albumin;
• and combinations thereof.

12. The pharmaceutical composition according to anyone of claims 10-11, formulated in the form of powder, granules, flakes, tablets, lozenges, briquettes, pills, liquid, semi-solid, gelled in the form of creams, ointments, lotions, gels, unguents, macroemulsions, microemulsions, nanoemulsions, serums, balms, colloids, solutions, liquids, suspensions, dispersions, spray formulations, suppositories, ovules.

13. The pharmaceutical composition according to anyone of claims 10-12, formulated for an administration chosen among topical, oral, pulmonary, intranasal, buccal, rectal, vaginal, parenteral, intravenous, intracutaneous, subcutaneous, intramuscular, ophthalmic.

14. The compound according to anyone of claims 1-9 or the pharmaceutical composition according to anyone of claims 10-13 for use as a medicament.

15. The compound or composition according to claim 14 for use in the treatment of cancers, preferably chosen from:
• colon carcinoma;
• colorectal carcinoma;
• hepatocellular carcinoma;
• carcinoma of the oral cavity or pharynx;
• basal cell carcinoma; and
• cervical carcinoma.

16. A medical device comprising at least one compound according to anyone of claims 1-9 or at least one composition according to anyone of claims 10-13, wherein said device is configured for the release over time of said at least one compound or of said at least one composition and is chosen from plasters, bandages, fabric cloths, wipes, masks, aerosols.
